(19) Europäisches Patentamt
European Patent Office
Office européen des brevets

(11) Veröffentlichungsnummer: **0 385 936 A1**

(12) # EUROPÄISCHE PATENTANMELDUNG

(21) Anmeldenummer: **90810121.5**

(22) Anmeldetag: **20.02.90**

(51) Int. Cl.[5]: **C12P 21/00, C12P 1/06, A61K 37/02, //(C12P1/06, C12R1:465)**

Der Anmelder hat eine Erklärung nach Regel 28 (4) EPÜ (Herausgabe einer Probe nur an einen Sachverständigen) eingereicht. Eingangsnummer(n) der Hinterlegung(en): DSM 5195.

(30) Priorität: **27.02.89 CH 707/89**

(43) Veröffentlichungstag der Anmeldung: **05.09.90 Patentblatt 90/36**

(84) Benannte Vertragsstaaten: **AT BE CH DE DK ES FR GB GR IT LI LU NL SE**

(71) Anmelder: **CIBA-GEIGY AG**
**Klybeckstrasse 141**
**CH-4002 Basel(CH)**

(72) Erfinder: **Zähner, Hans, Prof.Dr.**
**Im Hopfengarten 13**
**D-7400 Tübingen(DE)**
Erfinder: **Andres, Nikolaus, Dr.**
**Auf der Morgenstelle 28**
**D-7400 Tübingen(DE)**
Erfinder: **Zeeck, Axel, Prof.Dr.**
**Brüder-Grimm-Allee 22**
**D-3400 Göttingen(DE)**
Erfinder: **Rössner, Ellen, Dr.**
**Schiefer Weg 5a**
**D-3400 Göttingen(DE)**
Erfinder: **Wolf, Heinz, Prof. Dr.**
**Hohenlehenstrasse 12**
**D-7408 Wankheim(DE)**
Erfinder: **König, Wilfried A., Prof.Dr.**
**Bahnstrasse 95**
**D-2056 Glinde(DE)**
Erfinder: **Sinnwell, Volker, Dr.**
**Francoper Strasse 90a**
**D-2105 Hamburg 92(DE)**
Erfinder: **Fredenhagen, Andreas, Dr.**
**Spalenvorstadt 35**
**CH-4051 Basel(CH)**

(54) **Peptidlacton und derivate davon, Verfahren zu ihrer Herstellung und Mittel zur Ausführung des Verfahrens.**

(57) Beschrieben sind die Substanz Hormaomycin und daraus durch Lactonspaltung und gegebenenfalls nachfolgende Veresterung mit einem Niederalkanol erhältliche Verbindungen. Hormaomycin ist durch Fermentation mit dem Stamm Streptomyces griseoflavus DSM 5195 erhältlich und weist gegenüber den Stämmen Arthrobacter crystallopoietes ATCC 15481 und Arthrobacter oxydans ATCC 14358 eine minimale Hemmkonzentration auf, welche kleiner als 0,001 µg/ml ist. Hormaomycin kann als Arzneimittel und zur Anregung der Luftmycelbildung bei Streptomyceten verwendet werden und besitzt wahrscheinlich die in Formel I wiedergegebene Struktur.

EP 0 385 936 A1

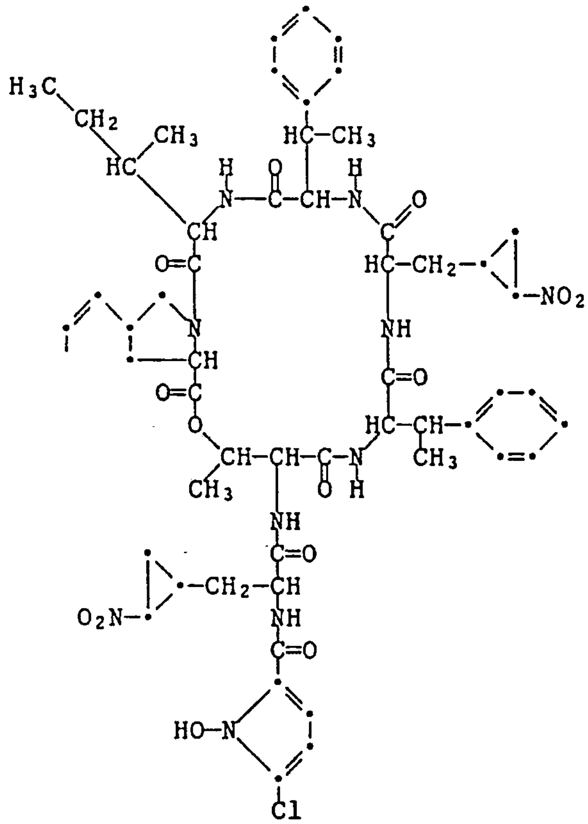

(I)

## Peptidlacton und Derivate davon, Verfahren zu ihrer Herstellung und Mittel zur Ausführung des Verfahrens

Die Erfindung betrifft ein Fermentierungsprodukt aus Streptomyces griseoflavus, welches die Struktur eines Peptidlactons besitzt, ein Fermentierungsverfahren zu seiner Herstellung, aus dem Peptidlacton durch Lactonspaltung und gegebenenfalls nachfolgende Veresterung mit einem Niederalkanol erhältliche Derivate, die Verwendung des Peptidlactons und der genannten Derivate als Arzneimittel und zur Anregung der Luftmycelbildung und Antibiotikaproduktion bei Streptomyceten, pharmazeutische Präparate, welche das Peptidlacton oder eines der genannten Derivate enthalten, neue Abbauprodukte und den neuen Produzentenstamm.

Bakterien der Gattung Streptomyces sind durch einen komplexen Entwicklungszyklus gekennzeichnet, bei dem sich auf festem Nährboden aus einem Substratmycel ein Luftmycel bildet. In einem Sporulationsprozess können dann endständige Luftmycelhyphen in Sporenketten fragmentieren. Experimentelle Befunde lassen vermuten, dass die Zelldifferenzierung in Luftmycel und Sporen während des sekundären Stoffwechsels stattfindet, also gleichzeitig mit der Synthese von Antibiotika, Pigmenten, Speicherstoffen und Geosminen.

Ueber die Regulation des Sekundärstoffwechsels und der Differenzierung bei Streptomyceten ist bisher wenig bekannt. Beide Prozesse scheinen aber durch zwei Gruppen von Signalsubstanzen kontrolliert zu werden, die entweder in der Zelle oder zwischen den Zellen vermitteln.

Kürzlich wurde bei Streptomyceten gezeigt, dass sowohl die Luftmycelentwicklung als auch die Antibiotikasynthese stringent kontrolliert werden. Die stringente Kontrolle wird durch zwei intrazelluläre Effektoren ausgelöst, Guanosin-tetra- und Guanosin-penta-phosphat.

Neben diesen bei Bakterien allgemein verbreiteten Effektoren sind interzelluläre, stammspezifische Signalmoleküle gefunden worden. Sie induzieren bei Actinomyceten Luftmycel- und/oder Antibiotikabildung wie z.B. der A-Faktor (2-Isooctanoyl-3(R)-hydroxymethyl-γ-butyrolacton) und der B-Faktor (3′-(1-Butylphosphoryl)-adenosin).

Auf der Suche nach neuen interzellulären Signalsubstanzen wurde nun mit Hilfe des Agardiffusionstests ein Stamm gefunden, der eine Substanz mit der gewünschten Aktivität produziert. Die Substanz regt in geringer Konzentration die Luftmycel- und Antibiotikabildung beim Produzentenstamm und bei mehreren anderen Streptomyceten-Stämmen an und wird deshalb Hormaomycin genannt (griechisch hormao = ich rege an).

Die Erfindung betrifft die Substanz Hormaomycin, welche durch Fermentation mit dem Stamm Streptomyces griseoflavus DSM 5195 erhältlich ist und gegenüber den Stämmen Arthrobacter crystallopoietes ATCC 15481 und Arthrobacter oxydans ATCC 14358 eine minimale Hemmkonzentration aufweist, welche kleiner als 0,01, ja sogar kleiner als 0,001 µg/ml ist.

Hormaomycin besitzt nach den bisherigen Befunden wahrscheinlich die Struktur der Formel I.

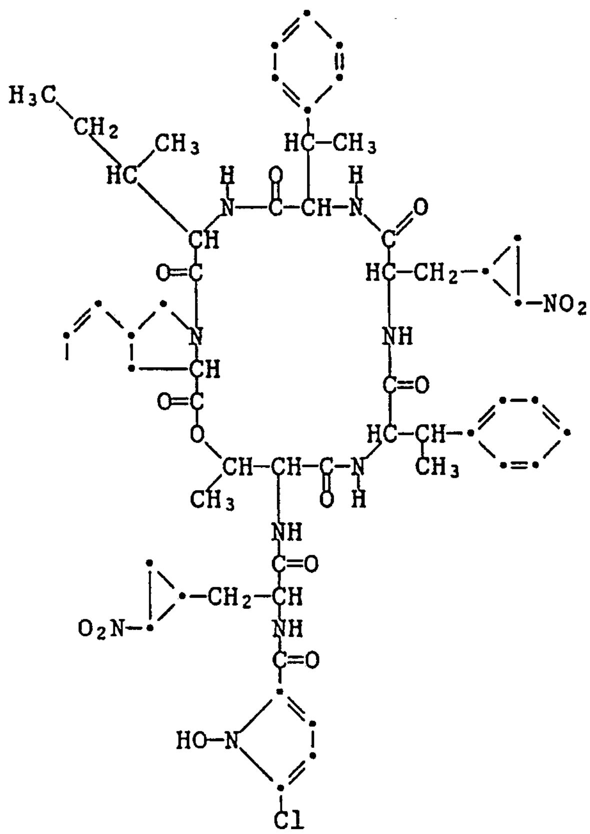

(I)

Die Erfindung betrifft insbesondere Hormaomycin, welches die im Beispielteil angegebenen Merkmale aufweist, soweit diese als gesichert gelten können, wobei zur eindeutigen Charakterisierung bereits eine Auswahl dieser Merkmale genügt, das heisst z.B. Hormaomycin, welches innerhalb der Messgenauigkeit mindestens eine oder vorzugsweise alle der folgenden physikalischen Eigenschaften aufweist:

a) Schmelzpunkt 166-168° C,

b) optische Drehung $[\alpha]_D^{20}$ = +20,8° (c = 0,5; Methanol),

c) Molekulargewicht 1129±2,

d) in $CDCl_3$ als Lösungsmittel $^1H$-NMR-Signale mit einer chemischen Verschiebung $\delta$ gegenüber Tetramethylsilan als interner Standard zwischen +0,5 und -0,7 ppm, insbesondere Signale für je ein Proton bei 0,48, 0,29, -0,15 und -0,63 ppm.

Besonders charakteristisch für Hormaomycin ist auch, dass es 2 Reste der Aminosäure 2-Amino-3-(2-nitro-cyclopropyl)-propionsäure aufweist, die im folgenden auch als 3-(2-Nitro-cyclopropyl)-alanin (abgekürzt NCP-Ala) bezeichnet wird, wobei sich diese beiden Reste hinsichtlich der Konfiguration an den Asymmetriezentren voneinander unterscheiden können.

Nachfolgend und im Beispielteil werden die Befunde geschildert, aus denen auf die Struktur der Formel I geschlossen werden kann:

Das optisch aktive Hormaomycin zeigt typische UV-Maxima bei 278 und 206 nm (Methanol), die sich bei pH-Aenderungen nur wenig verschieben. Im IR-Spektrum sind Banden für CO-Lacton (1741 $cm^{-1}$) und CO-Amid (um 1640 und 1545 $cm^{-1}$) zu erkennen. Die Molmasse von 1128,4 ergibt sich aus dem FAB-Massenspektrum. Der Elementaranalyse nach enthält Hormaomycin neben C, H, N und O auch ein Chloratom, entsprechend der Bruttoformel $C_{55}H_{69}N_{10}O_{14}Cl$.

Das $^1H$-NMR-Spektrum in $CDCl_3$ zeigt sechs deutlich unterscheidbare, mit $CD_3OD$ zum Teil langsam austauschende NH-Gruppen und eine OH-Gruppe bei 11,1 ppm. Zehn eng beieinander liegende Aromat-H sowie ein AB-System (6,75/6,08 ppm, J = 4,5) sind zu erkennen. Im Bereich 2-5,5 ppm liegen zahlreiche 1H-Signale gut voneinander getrennt. Eine Signalhäufung tritt zwischen 1-2 ppm auf, erkennbar sind fünf Dubletts und ein Triplett für Methylgruppen. Bemerkenswert und für das Hormaomycin typisch sind zwei 1H-Multipletts bei 0,48 und 0,29 ppm sowie zwei weitere jenseits von Tetramethylsilan (TMS) bei -0,15 und -0,63 ppm.

Im $^{13}C$-NMR-Spektrum geben sich insgesamt 55°C-Atome zu erkennen, die folgende Qualität haben: 6 $CH_3$, 7 $CH_2$, 16 $sp^3$-CH, 14 $sp^2$-CH, 4 $sp^2$-C und 8 CO. Im Bereich der $sp^3$-C-Atome fallen zwei

Methylgruppen (13,4 ppm) übereinander, und im Bereich der $sp^2$-CH sind vier Signale (127,4, 127,7, 128,5, 128,6 ppm) und im Bereich der CO-Gruppen ist eine CO-Gruppe (168,7 ppm) doppelt zu zählen. Das Spektrum lässt vermuten, dass Hormaomycin ein Peptid ist, das zwei aromatische und weitere ungesättigte Aminosäuren enthält.

Aminosäure-Bausteine

Die im Hormaomycin enthaltenen Aminosäuren ergeben sich durch Identifizierung der bei der Hydrolyse erhaltenen Komponenten und durch Zuordnung im intakten Antibiotikum mit Hilfe von $^1H/^1H$- und $^1H/^{13}C$-COSY-NMR-Spektren.

Im Totalhydrolysat von Hormaomycin (6 M HCl-Essigsäure, 1:1, 110 °C, 48 Stunden) lassen sich mit der automatischen Aminosäureanalyse und durch Mikrodansylierung [H. Laatsch, J. Chromatogr. 173 (1979) 398] Threonin (Thr) und Isoleucin (Ile) nachweisen. Ungewöhnliche, neutrale Aminosäuren sind im Bereich des Prolins, des Valins (zwei Banden) und des Phenylalanins (Bande mit doppelter Intensität) zu erkennen. Für die Untersuchung in der GC-MS-Kopplung (Gaschromatograph, der mit einem Massenspektrometer verbunden ist) überführt man die Aminosäuren in die trifluoracetylierten Methylester bzw. Isopropylester oder stellt Trimethylsilylderivate her. Aus den Massenspektren der Substanzen mit kleinster Retentionszeit können a-Thr und Ile zugeordnet werden. Ferner lässt sich die doppelt vorkommende Aminosäure als $\beta$-Methyl-phenylalanin ($\beta$-Me-Phe) durch die charakteristischen Fragmente bei m/z 230 ($M^+$-$COOCH_3$) und 105 ($[C_6H_5CHCH_3]^+$) im EI-MS (Elektronenstoss-Ionisation-Massenspektrum) des trifluoracetylierten Methylesters und das Molekülion bei m/z 318 ($[M + H]^+$) im CI-MS (Chemische Ionisation-Massenspektrum) des trifluoracetylierten Isopropylesters zuordnen. Die Daten stimmen mit denen für synthetisches $\beta$-Me-Phe überein [Y. Kataoka et al., Bull. Chem. Soc. Jap. 49 (1976) 1081]. Für die bisher genannten Amminosäuren können, ausgehend von den Methylgruppen bzw. von den NH- und $\alpha$-CH-Gruppen, die zusammengehörenden Kopplungspartner im $^1H/^1H$-COSY-Spektrum aufgefunden und über das $^1H/^{13}C$-COSY-Spektrum mit den zugehörigen C-Atomen verknüpft werden. Die Signale der beiden $\beta$-Me-Phe-Bausteine unterscheiden sich zum Teil deutlich. Auffällig ist ferner, dass die $H\alpha/H\beta$-Kopplung einmal J = 4,5 Hz wie in der freien Aminosäure, und einmal J = 10,5 Hz beträgt, was auf unterschiedliche Konformationen der Seitenketten hindeutet.

Als eine von drei neuen Aminosäuren wird (Z)-4-Propenyl-prolin (4-PE-Pro) identifiziert. Den Hinweis auf die Molmasse bzw. das Prolingerüst liefert das Ion bei m/z 265 ($M^+$) des trifluoracetylierten Methylesters in Verbindung mit den Ionen bei m/z 206 ($M^+$-$COOCH_3$) und 164. Die Struktur der Seitenkette und ihre Position am Prolingerüst ergibt sich aus den Korrelationsspektren. H-C(4) und $H_2$-C(5) (s. Tabelle 1) überlappen in $CDCl_3$ und sind im $^1H/^1H$-COSY-Spektrum nicht zweifelsfrei zuzuordnen (3,21 und 3,21/3,91 ppm). In $CDCl_3/(CD_3)_2CO/C_6D_6$6 (1:1:1) hingegen kann eine genaue Zuordnung getroffen werden (2,98 und 3,07/3,85 ppm). Das olefinische AB-System ($\delta_H$ 5,20/5,58; $\delta_C$ 127,5/128,2 in $CDCl_3$) besitzt eine AB-Kopplung von J = 10,5 Hz (Z-Konfiguration) und wird auf der einen Seite durch eine olefinische Methylgruppe ($\delta_H$ 1,61), auf der anderen durch eine CH-Gruppe weiter aufgespalten. Ausgehend von $\alpha$-CH (4,20 ppm) kommt man über eine $CH_2$- (1,72/2,30 ppm) und eine CH- (3,21 ppm) zu einer weiteren $CH_2$-Gruppe (3,21/3,91 ppm). Letztere entspricht mit ihrem $\delta_C$ -Wert (52,8 ppm) dem C(5) des Prolins, was die 4-Position des Propenylrestes am Prolingerüst beweist. In der automatischen Aminosäureanalyse liegt der Anteil von 4-PE-Pro immer deutlich unter 1. Man kann bei der GC-MS-Analyse neben dem N-Trifluoracetyl-4-propenyl-prolinmethylester noch entsprechende 4-(1-oder 2-Trifluoracetoxypropyl)- bzw. 4-(1- oder 2-Chlorpropyl)-Derivate an Hand der Massenspektren nachweisen. Dies erklärt sich mit der Annahme, dass 4-PE-Pro bei der Hydrolyse des Hormaomycins Wasser bzw. HCl an die Doppelbindung addiert.

Die beiden noch nicht zugeordneten Aminosäuren des Totalhydrolysates geben als trifluoracetylierte Methylester in der GC-MS-Kopplung identische EI-Massenspektren, die sich keiner bekannten Verbindung zuordnen lassen. Um diese Aminosäuren genauer untersuchen zu können, wird das Totalhydrolysat von 10 mg Hormaomycin einer HPLC-Trennung an einer $\mu$-Bondapak-$NH_2$-Säule (mit $NH_2$-Gruppen belegtes Kieselgel) im System Acetonitril-Wasser (7:3) unterworfen. Die Trennung gelingt nicht vollständig, in einer Fraktion jedoch sind der automatischen Aminosäureanalyse zufolge die unbekannten Aminosäuren und Isoleucin (Ile) jeweils in etwa gleichen Mengen enthalten. Erstere geben nach Derivatisierung wiederum identische EI-Massenspektren und liefern im CI-MS das Molekülion bei m/z 285 $[M + H]^+$. Die geradzahlige Molmasse spricht für zwei N-Atome im Molekül, was durch Hochauflösung der Fragment-Ionen im EI-MS und des Molekülions der Trimethylsilyl-Derivate bewiesen wird und zur Summenformel $C_6H_{10}N_2O_4$ für die freien Aminosäuren führt. Der trifluoracety lierte Methylester verliert als erstes $NO_2$ (m/z 238) oder $COOCH_3$ (m/z 225) und zeigt weitere typische Fragment-Ionen einer Nitroaminosäure. Bei der Suche nach den

zugehörigen $^1H$-NMR-Signalen in den Korrelationsspektren des Hormaomycins stösst man, ausgehend von den NH/α-CH-Gruppen, auf zwei identische Korrelationsreihen, von denen eine leicht auszumachen ist, die andere sich aufgrund von Signalüberlappungen erst durch Kombination von $^1H/^1H$-COSY- und $^1H/^{13}C$-COSY-Spektren bei 400 MHz ergibt (Tabelle 1). MS- und NMR-Daten beweisen, dass 3-(2-Nitro-cyclopropyl)-alanin (NCP-Ala) vorliegt, welches zweimal im Hormaomycin enthalten ist. Während sich die entsprechenden $^1H$-NMR-Signale der beiden Bausteine im intakten Peptid deutlich ($\Delta\delta$ = 0,82 bis 1,67 ppm) unterscheiden, sind die vergleichbaren $^{13}C$-NMR-Signale sehr ähnlich ($\Delta\delta < 2$ ppm, Tabelle 1). Die für das Hormaomycin typischen Hochfeld-verschobenen $^1H$-NMR-Signale gehören zu einem NCP-Ala. Für die Abschirmung könnten die Phenylreste der β-Me-Phe-Bausteine verantwortlich sein.

Bei gleicher Konstitution kann die Trennbarkeit der freien Aminosäure in der automatischen Aminosäureanalyse und nach Derivatisierung bei der GC-MS-Analyse nur in der Stereochemie begründet sein. H-C(5) (2,90 und 3,99 ppm) ist in beiden Bausteinen nicht verdeckt und zeigt ein identisches Kopplungsmuster (ddd, 5-Linien), aus dem sich die Kopplungskonstanten (J = 6,6, 3,3 und 3,3) ableiten lassen. Aus der Grösse der Kopplungskonstanten von H-Atomen am Cyclopropanring [H. Fritschi et al., Helv. Chim. Acta 71 (1988) 1553] folgt, dass auf H-C(5) nur eine cis-Kopplung zu einem der beiden Protonen an C-(6) liegt und die Nitrogruppe und der Alanylrest somit trans zueinander am Cyclopropanring stehen. Das H-C(5)-Kopplungsmuster kehrt in gleicher Weise im $^1H$-NMR-Spektrum der freien, HPLC-angereicherten Aminosäure NCP-Ala auf dem Signal bei 4,40 ppm wieder, das trotz des Vorliegens von Stereoisomeren nicht doppelt auftritt. Vermutlich unterscheiden sich die beiden Aminosäuren in der Konfiguration am α-C-Atom.

Fügt man die im Totalhydrolysat des Hormaomycins nachgewiesenen Aminosäuren unter Kondensation zusammen, dann sind an der Peptidkette ein Aminoende, ein Carboxylende sowie die a-Threonin-OH-Gruppe (allo-Threonin-OH-Gruppe) frei und es fehlt noch ein $C_5N$-Rest, der neben H- und O-Atomen auch das nachgewiesene Cl-Atom enthalten muss. Das Aminoende der Peptidkette ist acyliert, nur so wird die Zahl von insgesamt sechs Amid-NH-Gruppen im $^1H$-NMR-Spektrum erreicht. Dementsprechend verlaufen Endgruppenbestimmungen mit 2,4-Dinitro-fluorbenzol und Dansylchlorid negativ. Die a-Threonin-OH-Gruppe ist verestert, erkennbar an der Tieffeldlage von H-C(3) (5,35 ppm). Dass der $C_5N$-Rest nicht als Ester sondern amidisch an die Peptidkette gebunden ist, folgt aus der Lactonspaltung von Hormaomycin mit Methanol/$K_2CO_3$ [W.A. Ayer und L.M. Pena-Rodriguez, J. Nat. Prod. 50 (1987) 400]. Unter Aufnahme von Methanol entsteht ein Hormaomycin-methylester mit der Molmasse 1160 (Fast atom bombardment-Massenspektrum, FAB-MS): 1161 $[M + H]^+$, 1183 $[M + Na]^+$), in dessen $^1H$-NMR-Spektrum H-C(3) des Threonins auf $\delta_H < 5$ ppm verschoben ist und ein Ester-$OCH_3$ bei 3,74 ppm auftaucht. Der noch nicht zugeordnete Molekülteil besteht somit aus einer CO-Gruppe $\delta_C$ 159,3 ppm) und einem Rest $C_4H_3NOCl$. Die C-Atome sind $sp^2$-hybridisiert mit $\delta_C$-Werten zwischen 103,5 und 121,4 ppm. Zwei der H-Atome bilden das AB-System bei $\delta_H$ 6,08/6,75 ppm, das dritte liegt bei 11,10 (Tabelle 1). Diese Daten lassen sich plausibel zur amidisch gebundenen, in der Literatur unbekannten 5-Chlor-N-hydroxy-pyrrol-2-carbonsäure (CHPCA) zusammenfügen, die in den Hydrolysaten des Hormaomycins bisher nicht nachgewiesen werden konnte. Die NMR-Daten (Tabelle 1) stimmen mit denen für in 5-Stellung substituierte Pyrrol-2-carbonsäuren sowie denen für vergleichbare Reste in den Antibiotika Chromoxymycin [Y. Kawai et al., Tetrahedron Lett. 26 - (1985) 3273] und 55185 RP [ M. Dechamps et al., Europäische Patentanmeldung 246 975] befriedigend überein.

Im Hormaomycin bilden die nachgewiesenen Aminosäuren und die postulierte Pyrrol-2-carbonsäure über Amidbindungen eine lineare Peptidkette, deren Carboxylende mit der a-Threonin-OH-Gruppe einen Lactonring schliesst. Der Lactonring trägt am a-Threonin eine amidisch gebundene Seitenkette.

Die postulierte Sequenz der Aminosäurebausteine im Lactonring ergibt sich aus folgenden Untersuchungen:

Wie oben beschrieben, ist a-Threonin mit seiner Hydroxyfunktion an der Lactonbindung beteiligt, was sich aus $^1H$-NMR-Daten erkennen lässt.

Die Aminosäure, die ihre Carboxyfunktion für die Lactonbindung zur Verfügung stellt, sollte durch Reduktion der Esterfunktion zum Alkohol bestimmbar sein. Bei der Umsetzung von Hormaomycin mit $LiAlH_4$ in Tetrahydrofuran bei 0° C, kann nach Totalhydrolyse des Reduktionsproduktes und anschliessender Derivatisierung zu den trifluoracetylierten Methylestern das entsprechende 4-Propenyl-prolinol-Derivat in der GC-MS-Analyse nachgewiesen werden.

N,O-Bis-trifluoracetyl-4-propenyl-prolinol:

EI-MS: m/z = 333 (10 %, $M^+$), 206 (100 %, $M^+$ - $[CH_2$-$OCOCF_3]$), 164 (10 %, $M^+$ - $[CH_2$-$OCOCF_3$ + $CH=CH$-$CH_3$ + $H]$).

Durch Oeffnung des Lactonringes von Hormaomycin und Bildung des Methylesters erhält man die als Hormaomycin-methylester bezeichnete Verbindung der Formel II.

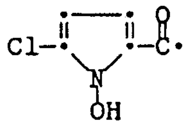•NCP-Ala•a-Thr•β-Me-Phe•NCP-Ala•β-Me-Phe•Ile•4-PE-Pro-$OCH_3$

(II)

Die Erfindung betrifft auch die aus Hormaomycin durch Lactonspaltung und Veresterung mit einem Niederalkanol erhältlichen Substanzen, insbesondere Hormaomycin-methylester.

Eine vergleichende Analyse der FAB-Massenspektren von Hormaomycin und dem offenkettigen Methylester der Formel II ergibt folgendes:

pos. FAB-MS von Hormaomycin:

m/z = 1129,4/1131,5 (11 %, $[M+H]^+$], 938 (1 %, $[M+H]^+$ -(CHPCA + $HNO_2$)), 830 (2 %, $[M+2H]^+$ -(NCP-Ala•CHPCA)), 700/702 (2 %, $[M+2H]^+$ -(NCP-Ala•β-Me-Phe•Ile)), 538/540 (1 %, $[M+H]^+$ -(β-Me-Phe•NCP-Ala•β-Me-Phe•Ile)), 516/518 (2 %, $[M+H]^+$ -(NCP-Ala•β-Me-Phe•Ile°&4-PE-Pro + $NO_2$)), 401/403 (2 %, $[M+H]^+$ -(β-Me-Phe•NCP-Ala•β-Me-Phe•Ile•4-PE-Pro)), 300/302 (2 %, NCP-Ala•CHPCA), 144/146 (22 %, CHPCA, 134 (100 %).

pos. FAB-MS der Verbindung der Formel II:

m/z = 1183/1185 (3,5 %, $[M+Na]^+$, 1161/1163 (2 %, $[M+H]^+$), 992/994 (3,6 %, $[M+H]^+$ - (4-PE-Pro-OMe + H)), 879/881 (1 %, $[M+H^+$ -(Ile•4-PE-Pro-OMe + H)), 718/720 (3 %, $[M+H]^+$ - (β-Me-Phe•Ile•4-PE-Pro-OMe + H)), 562/564 (0,9 %, $[M+H]^+$ -(NCP-Ala•β-Me-Phe•Ile•4-PE-Pro-OMe + H)), 534/536 (0,7 %, $[M+H]^+$ - (OC-NCP-Ala•β-Me-Phe•Ile•4-PE-Pro-OMe + H)), 419 (3 %, a-Thr•β-Me-Phe•NCP-Ala + H), 401/403 (0,9 %, a-Thr•β-Me-Phe•NCP-Ala + H).

Massenzahlen der Aminosäurebausteine CO-CHR-NH:

a-Thr : 100 bzw. 101
Ile : 113
β-Me-Phe: 161
4-PE-Pro: 137
NCP-Ala : 156
CHPCA : 144

Auch das FAB-MS der Verbindung der Formel II zeigt eine Abspaltung des 4-PE-Pro-$OCH_3$-Restes vom Carboxyl-Ende des offenkettigen Peptids, was die Beteiligung an der Lactonbindung im Hormaomycin bestätigt.

Bei einer Partialhydroylse von Hormaomycin (12N HCl/Essigsäure 1:1, Raumtemperatur, 48 Stunden) können nach Derivatisierung zu den trifluoracetylierten Methylestern zwei Dipeptide und ein Tripeptid in der GC-MS-Analyse aufgefunden werden.

-N-Trifluoracetyl-β-methyl-phenylalanin-isoleucin-methylester:

EI-MS: m/z = 402 (5 %, $M^+$), 343 (2 %, $M^+$-$COOCH_3$), 230 (5 %, $M^+$ -(OC-Ile-$OCH_3$)), 105 (100 %, $C_6H_5$-CH-$CH_3$).

-N,O-Bis-trifluoracetyl-allo-threonin-β-methyl-phenylalanin-methylester:

EI-MS: m/z = 427 (0,3 %, $M^+$-$COOCH_3$), 266 (0,2 %, $M^+$ -(OC-β-Me-Phe-$OCH_3$)), 192 (1,5 %, β-Me-Phe-$OCH_3$), 176 (16 %, $C_6H_5$-$C(CH_3)$=CH-$COOCH_3$), 134 (9 %, $NH_2$-CH-$CH(CH_3)$-$C_6H_5$), 105 (100 %, $C_6H_5$-CH-$CH_3$).

- N-Trifluoracetyl-β-methyl-phenylalanin-isoleucin-4-propenyl-prolin-methylester:

EI-MS: m/z = 539 (1 %, $M^+$), 483 (3 %, $M^+$ - ($C(CH_3)$=$CH(CH_3)$), 343 (5 %, $M^+$ - (OC-4-PE-Pro-$OCH_3$)), 230 (1 %, $M^+$ -(OC-Ile•4-PE-Pro-$OCH_3$), 105 (96 %, $C_6H_5$-CH-$CH_3$).

Diese Ergebnisse bestätigen Teile der Sequenzanalyse aus den FAB-MS-Spektren.

Die Stereochemie der Aminosäuren und die Konformation des Lactonringes ist noch zum Teil offen.

Hormaomycin zeigt in vitro in einem Agar-Verdünnungsreihentest eine antimikrobielle Wirkung gegen einige Bakterien, z.B. Arthrobacter crystallopoites, Arthrobacter oxydans, Corynebacterium species, Brevibacterium linens und Salmonella typhimurium. Die minimale Hemmkonzentration liegt im Bereich zwischen 0,0001 und 50 µg/ml. Die nachfolgende Tabelle offenbart einige spezifische Beispiele:

| Stamm | Minimale Hemmkonzentration (µg/ml) |
|---|---|
| Arthrobacter crystallopoietes ATCC 15481* | 0,0001 |
| Arthrobacter oxydans ATCC 14358 | 0,0005 |
| Corynebacterium sp. ATCC 23830 | 0,1 |
| Brevibacterium linens ATCC 9172 | 10 |
| Salmonella typhimurium ATCC 13311 | 50 |

* ATCC: American Type Culture Collection, Rockville, MD, USA

Daher kann Hormaomycin als antimikrobielles Mittel verwendet werden. Bei Verwendung als Antibiotikum im Falle einer Infektion durch Salmonella typhimurium beträgt die an einen Warmblüter von etwa 70 kg Körpergewicht täglich zu verabreichende Dosis etwa 0,5-5 g, vorzugsweise 1-2 g.

Hormaomycin bewirkt bereits in einer Konzentration ab etwa 0,05 µg/ml bei einigen Streptomyceten-Stämmen eine Erhöhung der Bildung von Antibiotika. Zum Beispiel bewirkt die Zugabe von Hormaomycin in einer Konzentration von 0,05 µg/ml zu einer Fermentationsbrühe, enthaltend Streptomyces griseoflavus (Tü 1306), eine Verdoppelung und in einer Konzentration von 1 µg/ml eine ungefähre Vervierfachung der Produktion des Antibiotikums Tirandamycin.

Hormaomycin kann daher auch zur Steigerung der Ausbeute an wertvollen Fermentationsprodukten verwendet werden.

Hormaomycin induziert die Luftmycelbildung von Streptomyces griseoflavus (DSM 5195) und von weiteren Streptomyces-Stämmen. Verwendet man diese Stämme als Indikatorbakterien für Testplatten und zusätzlich einen Nähragar, auf dem sich kein Luftmycel (erkennbar an der weissen Färbung) entwickelt, so vermag 1 µg Hormaomycin, in einer Papierrondelle auf die Agaroberfläche aufgebracht, reichliche Luftmycelbildung um die Rondelle herum auszulösen. Zusätzlich induziert die Substanz die Antibiotikaproduktion in diesen Stämmen.

Das erfindungsgemässe Methodikverfahren zur Herstellung von Hormaomycin ist dadurch gekennzeichnet, dass man den Stamm Streptomyces griseoflavus DSM 5195 oder eine von diesem Stamm ableitbare Kultur in einem geeigneten Nährmedium züchtet und Hormaomycin aus der Kulturbrühe isoliert.

Das obige Verfahren wird im folgenden näher erläutert: Hormaomycin wird mit Hilfe eines neuen mikrobiologischen Verfahrens, nämlich durch Fermentation mit dem neuen Stamm Streptomyces griseoflavus DSM 5195, erhalten, welcher am 31. Januar 1989 bei der DSM Deutsche Sammlung von Mikroorganismen und Zellkulturen, Mascheroder Weg 1B, D-3300 Braunschweig, Bundesrepublik Deutschland, unter der Nummer DSM 5195 hinterlegt wurde.

Der Produzentenstamm des Hormaomycins DSM 5195 wurde aus einer bei Anuradhapura (Sri Lanka) gesammelten Erdprobe isoliert. Er gehört zur Gattung Streptomyces, stimmt in allen artbestimmenden Merkmalen mit dem Typusstamm von Streptomyces griseoflavus überein und ist daher dieser Art zuzuordnen.

Der Stamm DSM 5195 ist durch folgende Merkmale gekennzeichnet (Nomenklatur und Systematik nach R. Hütter, "Systematik der Streptomyceten", Verlag Karger, Basel 1967): Die Sporen sind stachelig. Das Luftmycel ist anfangs kreideweiss und wird in ausgereiftem Zustand aschgrau (cinereus). Das Luftmycel ist monopodial verzweigt und besitzt Seitenäste mit mehr oder weniger regelmässigen Schrauben von meist 4-6 Windungen (Spira-Typ b). Auf Pepton-Eisen-Agar bildet der Stamm kein Melanin. Der Stamm verwertet die folgenden Zucker bzw. Alkohole als einzige C-Quelle: D-Fructose, D-Glucose, myo-Inosit, D-Mannose, L-Rhamnose und besonders gut D-Xylose. Auf L-Arabinose, Raffinose und Saccharose wird kein Wachstum beobachtet.

Als ableitbare Kultur im obigen Sinne gilt jede Kultur, welche noch die für die Durchführung des erfindungsgemässen Verfahrens wesentlichen Merkmale der hinterlegten Kultur aufweist, d.h. hauptsächlich eine abgeleitete Kultur, insbesondere eine Kultur, deren Mikroorganismen die gleichen Strukturgene wie die für die Bildung von Hormaomycin ursächlichen Strukturgene des Stammes DSM 5195 enthalten. Als ableitbare Kultur gilt auch jede Kultur, deren Mikroorganismen ein wegen der Entartung des genetischen Codes mögliches Aequivalent der für die Bildung von Hormaomycin ursächlichen Strukturgene des Stammes DSM 5195 enthalten. Als ableitbare Kultur gilt insbesondere jede Kultur, die Mikroorganismen der Gattung Streptomyces, vorzugsweise der Art Streptomyces griseoflavus, welche zur Produktion von Hormaomycin befähigt sind, enthält. Der Begriff "ableitbare Kultur des Stammes DSM 5195" schliesst auch alle

Mutanten dieses Stammes ein, die zur Produktion von Hormaomycin befähigt sind.

Als Nährmedium verwendet man eine vorzugsweise wässrige Lösung oder Suspension, welche mindestens eine Kohlenstoffquelle und mindestens eine Stickstoffquelle, und vorzugsweise auch Mineralstoffe enthält. Als Kohlenstoffquelle sind beispielsweise zu nennen: Glycerin, assimilierbare Kohlenhydrate, wie Cyclitole, z.B. Mannit, Polysaccharide, z.B. Stärke, Disaccharide, z.B. Lactose und Saccharose, und Monosaccharide, vor allem Glucose und Fructose, sowie entsprechende Kohlenhydrat-haltige technische Rohstoffe, wie Zuckerrüben- oder Zuckerrohrmelasse. Als Stickstoffquelle seien genannt: Aminosäuren, insbesondere die in der Natur vorkommenden α-Aminosäuren, Peptide sowie Proteine und deren Abbauprodukte, wie Peptone und Tryptone, aber auch Ammoniumsalze und Nitrate, sowie entsprechende technische stickstoffhaltige Rohstoffe, wie Fleischextrakte, Kaseinhydrolysat sowie Hefeautolysat und -extrakt. Es kommen auch gemischte technische C- und N-Quellen in Betracht, wie verschiedene Pflanzensamen, die in Form von wässrigen Auszügen, Mehl oder Maische verwendet werden, z.B. von Bohnen, z.B. Sojabohnen, Getreidekörnern, z.B. Weizen, und insbesondere Mais ("Corn-steep liquor"); ferner auch Baumwollsamen und Malzextrakt. Neben Ammoniumsalzen, wie insbesondere Ammoniumchlorid, -sulfat oder -nitrat und weiteren Nitraten kann das Nährmedium als anorganische Salze Chloride, Carbonate, Sulfate und insbesondere Phosphate von Alkali- und Erdalkalimetallen, wie insbesondere von Natrium, Kalium, Magnesium und Calcium, sowie von Spurenelementen, wie Eisen, Zink und Mangan, enthalten.

Vorzugsweise verwendet man das im Beispiel 1 beschriebene Nährmedium.

Das Nährmedium wird sterilisiert und mit einer Kultur des Produktionsstammes unter Einhalten der üblichen Massnahmen eingeimpft.

Die Züchtung erfolgt aerob, also beispielsweise in ruhender Oberflächenkultur oder vorzugsweise submers unter Schütteln oder Rühren mit Luft oder Sauerstoff im Schüttelkolben oder in Fermentern. Als Temperatur eignet sich eine solche zwischen etwa 15° C und 40° C, insbesondere zwischen etwa 20° C und 30° C. Die Fermentationszeit liegt bevorzugterweise zwischen 1 und 3 Tagen. Bei einer bevorzugten Ausführungsform des erfindungsgemässen Verfahrens inkubiert man zunächst 8 Stunden bei 27° C und senkt dann die Temperatur auf 20° C. Die Temperaturänderung bewirkt eine Ausbeutesteigerung an Hormaomycin. Unter diesen Bedingungen erreicht die Bildung von Hormaomycin nach 45 Stunden ein Maximum, wie mit Hilfe des Agardiffusionstests und durch Hochdruckflüssigkeitschromatographie ersichtlich. Vorzugsweise kultiviert man in mehreren Stufen, d.h. man stellt zunächst eine oder mehrere Vorkulturen in flüssigem Nährmedium her, die dann in das eigentliche Produktionsmedium, z.B. im Verhältnis 1:20, überimpft werden.

Die Isolierung erfolgt auf physiko-chemischen Wege mittels an sich bekannter Trennungsmethoden, insbesondere durch Zentrifugieren, Filtrieren, Lösungsmittel-Extraktion, Fällung, Kristallisieren und Chromatographie, vor allem Adsorptions- und Verteilungschromatographie.

Hormaomycin befindet sich zu 98 % in den Zellen. Daraus wird es zunächst mit einem geeigneten Lösungsmittel, z.B. einem Alkohol, wie Methanol, extrahiert. Das erhaltene Rohprodukt wird durch Extraktion mit einem geeigneten Lösungsmittel, wie Essigsäureethylester, und aufeinanderfolgende Chromatographie an Kieselgel und einem modifizierten Dextrangel, z.B. Sephadex®LH-20, in verschiedenen Lösungsmitteln gereinigt.

Insbesondere betrifft die Erfindung ein Verfahren zur Herstellung von Hormaomycin, dadurch gekennzeichnet, dass man den Stamm Streptomyces griseoflavus DSM 5195 oder einen Mikroorganismus, der die gleichen Strukturgene enthält wie die für die Bildung von Hormaomycin ursächlichen Strukturgene des genannten Stammes, in einem wässerigen, eine Kohlenstoff- und Stickstoffquelle sowie anorganische Salze enthaltenden Nährmedium aerob züchtet und Hormaomycin isoliert.

In erster Linie betrifft die Erfindung eine Ausführungsform des obengenannten Verfahrens, die dadurch gekennzeichnet ist, dass man einen Hormaomycin bildenden Mikroorganismus der Art Streptomyces griseoflavus züchtet.

Eine bevorzugte Ausführungsform des obengenannten Verfahrens ist dadurch gekennzeichnet, dass man den Stamm Streptomyces griseoflavus DSM 5195 oder eine Hormaomycin bildende Mutante dieses Stammes züchtet.

Eine besonders bevorzugte Ausführungsform des obengenannten Verfahrens ist dadurch gekennzeichnet, dass man den Stamm Streptomyces griseoflavus DSM 5195 züchtet.

Vorzugsweise führt man die Fermentation unter den im Beispielteil beschriebenen Bedingungen durch.

Mikroorganismen, die die gleichen für die Bildung von Hormaomycin ursächlichen Strukturgene enthalten wie der Stamm DSM 5195, können z.B. durch Genmanipulation künstlich geschaffen werden, indem man in an sich bekannter Weise die entsprechenden Strukturgene aus dem Stamm DSM 5195 isoliert und an geeigneter Stelle in das Genmaterial eines geeigneten anderen Mikroorganismus einbaut. Geeignete Mikroorganismen sind solche, in die man die betreffenden Strukturgene nicht nur einbauen kann, sondern in

denen diese Strukturgene auch ausgedrückt werden und in denen das gebildete Hormaomycin nicht wieder abgebaut sowie, vorzugsweise, in die Fermentationsbrühe ausgeschieden wird. Solche geeigneten Mikroorganismen sind in erster Linie andere Stämme der Gattung Streptomyces, insbesondere solche der Art Streptomyces griseoflavus, sofern sie die obengenannten Strukturgene nicht bereits besitzen.

Nach Aufklärung der Nucleotidsequenz der obengenannten Strukturgene können diese auch synthetisch hergestellt werden. Dabei besteht aufgrund der Entartung des genetischen Codes die Möglichkeit, bestimmte Nucleotidsequenzen aus drei aufeinanderfolgenden Nucleotiden, sogenannte Codons, gegen andere Codons auszutauschen, die für die gleiche Aminosäure codieren.

Hormaomycin bildende Mutanten können zum Beispiel unter der Einwirkung von Ultraviolett- oder Röntgenstrahlen oder von chemischen Mutagenen, z.B. N-Methyl-N′-nitro-N-nitroso-guanidin, erzeugt und durch Selektion nach ihren spezifischen Eigenschaften in an sich bekannter Weise isoliert werden.

Die Erfindung betrifft auch ein Verfahren zur Herstellung eines Fermentationsgutes, das Hormaomycin in nachweisbarer Menge enthält und das dadurch gekennzeichnet ist, dass man den Stamm Streptomyces griseoflavus DSM 5195 oder eine von diesem Stamm ableitbare Kultur in einem Nährmedium züchtet und das Verfahren zur Isolierung oder Reinigung von Hormaomycin an geeigneter Stelle abbricht.

Die Erfindung betrifft auch neue Spalt- bzw. Abbauprodukte des Hormaomycins, insbesondere neue Aminosäuren, z.B. 2-Amino-3-(2-nitrocyclopropyl)-propionsäure, (Z)-4-Propenyl-prolin und 2-Amino-3-phenylbuttersäure, sowie 5-Chlor-N-hydroxy-pyrrol-2-carbonsäure und im Hormaomycin vorgebildete Peptidbruchstücke, enthaltend mindestens eine der vorstehend genannten neuen Säuren.

Diese Spaltprodukte können zur chemischen Synthese des Hormaomycins verwendet werden.

Die Erfindung betrifft insbesondere die durch Lactonspaltung von Hormaomycin und gegebenenfalls nachfolgende Veresterung mit einem Niederalkanol erhältlichen Verbindungen der Formel III,

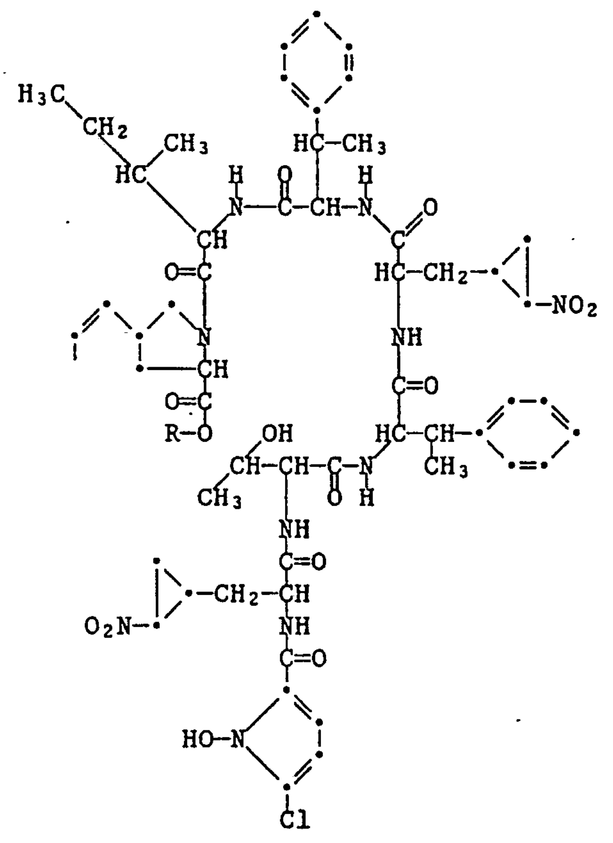

(III)

worin R für Wasserstoff oder Niederalkyl steht, und Salze der Verbindung der Formel III, worin R für Wasserstoff steht.

Salze einer Verbindung der Formel III sind vorzugsweise pharmazeutisch verwendbar und nicht toxisch, z.B. Alkalimetall- oder Erdalkalimetallsalze, z.B. Natrium-, Kalium-, Magnesium- oder Calciumsalze, oder Salze mit Ammoniak oder geeigneten organischen Aminen, wobei in erster Linie aliphatische, cycloaliphatische, cycloaliphatisch-aliphatische oder araliphatische primäre, sekundäre oder tertiäre Mono-, Di- oder Polyamine, sowie heterocyclische Basen für die Salzbildung in Frage kommen, wie Niederalkylamine, z.B.

Triethylamin, Hydroxyniederalkylamine, z.B. 2-Hydroxy-ethylamin, Bis-(2-hydroxyethyl)-amin, 2-Hydroxy-ethyl-diethylamin oder Tri-(2-hydroxyethyl)-amin, basische aliphatische Ester von Carbonsäuren, z.B. 4-Amino-benzoesäure-2-diethylaminoethylester, Niederalkylenamine, z.B. 1-Ethylpiperidin, Cycloalkylamine, z.B. Dicyclohexylamin, oder Benzylamine, z.B. N,N′-Dibenzylethylendiamin, ferner Basen vom Pyridintyp, z.B. Pyridin, Collidin oder Chinolin.

Zur Isolierung oder Reinigung können auch pharmazeutisch ungeeignete Salze Verwendung finden. Zur therapeutischen Anwendung gelangen jedoch nur die pharmazeutisch verwendbaren, nicht-toxischen Salze, die deshalb bevorzugt werden.

Die Erfindung betrifft auch ein Verfahren zur Herstellung einer Verbindung der Formel III oder eines Salzes der Verbindung der Formel III, worin R Wasserstoff bedeutet, welches dadurch gekennzeichnet ist, dass man Hormaomycin der Formel I einer Lactonspaltung unterzieht, wodurch man eine Verbindung der Formel III, worin R für Wasserstoff steht, oder ein Salz davon erhält, und zur Herstellung einer Verbindung der Formel III, worin R für Niederalkyl steht, gegebenenfalls die Verbindung der Formel III, worin R für Wasserstoff steht, erforderlichenfalls nach Schutz der freien Hydroxylgruppen durch leicht abspaltbare Schutzgruppen, verestert und vorhandene Schutzgruppen abspaltet, oder zur Herstellung eines Salzes die Verbindung der Formel III, worin R Wasserstoff bedeutet, in ein Salz überführt oder ein erhaltenes Salz in die freie Säure umwandelt.

Die Lactonspaltung wird in an sich bekannter Weise durchgeführt. Vorzugsweise behandelt man Hormaomycin in einem geeigneten Lösungsmittel mit einem geeigneten basischen Mittel, insbesondere einem schwach basischen Mittel, wie dem Salz einer starken Base und einer schwachen Säure, z.B. Kaliumcarbonat, Alkalicyanid oder Natriumhydrogensulfit. Wenn erwünscht, kann das basische Mittel in katalytischen Mengen eingesetzt werden. Wird die Lactonspaltung in einem geeigneten Niederalkanol, wie Methanol oder Ethanol, durchgeführt, erhält man einen Ester der Formel III, worin R für den diesem Niederalkanol entsprechenden Niederalkylrest steht.

Wenn die Lactonspaltung nicht in einem solchen Niederalkanol durchgeführt wird, kann die erhaltene Verbindung der Formel III, worin R für Wasserstoff steht, in an sich bekannter Weise verestert werden.

Die Veresterung kann z.B. so durchgeführt werden, dass man ein reaktionsfähiges Carbonsäurederivat einer Verbindung der Formel III, worin R für Wasserstoff steht, mit dem entsprechenden Alkohol umsetzt. Alternativ kann man die Verbindung der Formel III, worin R für Wasserstoff steht, durch Umsetzung mit einem reaktionsfähigen Derivat des als Veresterungskomponente gewünschten Alkohols verestern.

Geeignete Mittel zur Veresterung sind beispielsweise entsprechende Diazoniederalkane, z.B. Diazomethan, Diazoethan oder Diazo-n-butan. Diese Reagenzien werden in Gegenwart eines geeigneten inerten Lösungsmittels, wie eines aliphatischen, cycloaliphatischen oder aromatischen Kohlenwasserstoffs, wie Hexan, Cyclohexan, Benzol oder Toluol, eines halogenierten aliphatischen Kohlenwasserstoffs, z.B. Methylenchlorid, oder eines Ethers, wie eines Diniederalkylethers, z.B. Diethylether, oder eines cyclischen Ethers, z.B. Tetrahydrofuran oder Dioxan, oder eines Lösungsmittelgemisches, und, je nach Diazoreagens, unter Kühlen, bei Raumtemperatur oder unter leichtem Erwärmen, ferner, wenn notwendig, in einem geschlossenen Gefäss und/oder unter einer Inertgas-, z.B. Stickstoffatmosphäre, zur Anwendung gebracht.

Weitere geeignete Mittel zur Veresterung sind Ester entsprechender Alkohole, in erster Linie solche mit starken anorganischen oder organischen Säuren, wie Mineralsäuren, z.B. Halogenwasserstoffsäuren, wie Chlorwasserstoff-, Bromwasserstoff- oder Iodwasserstoffsäure, ferner Schwefelsäure, oder Halogen-schwefelsäure, z.B. Fluorschwefelsäure, oder starken organischen Sulfonsäuren, wie gegebenenfalls, z.B. durch Halogen, wie Fluor, substituierten Niederalkansulfonsäuren, oder aromatischen Sulfonsäuren, wie z.B. gegebenenfalls durch Niederalkyl, wie Methyl, Halogen, wie Brom, und/oder Nitro substituierten Benzolsulfonsäuren, z.B. Methansulfon-, Trifluormethansulfon- oder p-Toluolsulfonsäure. Solche Ester sind u.a. Niederalkylhalogenide, Diniederalkylsulfate, wie Dimethylsulfat, ferner Fluorsulfonsäureniederalkylester, z.B. Fluorsulfonsäuremethylester, oder gegebenenfalls Halogen-substituierte Methansulfonsäure-niederalkylester, z.B. Trifluormethansulfonsäuremethylester. Sie werden üblicherweise in Gegenwart eines inerten Lösungsmittels, wie eines gegebenenfalls halogenierten, wie chlorierten, aliphatischen, cycloaliphatischen oder aromatischen Kohlenwasserstoffs, z.B. Methylenchlorid, eines Ethers, wie Dioxan oder Tetrahydrofuran, oder eines Gemisches davon verwendet. Dabei wendet man vorzugsweise geeignete Kondensationsmittel, wie Alkalimetallcarbonate oder -hydrogencarbonate, z.B. Natrium- oder Kaliumcarbonat oder -hydrogencarbonat (üblicherweise zusammen mit einem Sulfat), oder organische Basen, wie üblicherweise sterisch gehinderte Triniederalkylamine, z.B. N,N-Diisopropyl-N-ethylamin (vorzugsweise zusammen mit Halogensulfonsäure-niederalkylestern oder gegebenenfalls halogensubstituierten Methansulfonsäureniederalkylestern) an, wobei unter Kühlen, bei Raumtemperatur oder unter Erwärmen, z.B. bei Temperaturen von etwa -20 °C bis etwa +50 °C, und, wenn notwendig, in einem geschlossenen Gefäss und/oder in einer Inertgas-, z.B. Stickstoffatmosphäre, gearbeitet wird.

Weitere Mittel zur Veresterung sind entsprechende trisubstituierte Oxoniumsalze (sogenannte Meerweinsalze), oder disubstituierte Carbenium-oder Haloniumsalze, worin die Substituenten die verethernden Reste sind, beispielsweise Triniederalkyloxoniumsalze, sowie Diniederalkoxycarbenium-oder Diniederalkylhaloniumsalze, insbesondere die entsprechenden Salze mit komplexen, fluorhaltigen Säuren, wie die entsprechenden Tetrafluorborate, Hexafluorphosphate, Hexafluorantimonate, oder Hexachlorantimonate. Solche Reagentien sind z.B. Trimethyloxonium- oder Triethyloxonium-hexafluorantimonat, -hexachlorantimonat, -hexafluorphosphat oder -tetrafluorborat, Dimethoxycarbeniumhexafluorphosphat oder Dimethylbromoniumhexafluorantimonat. Man verwendet diese Mittel vorzugsweise in einem inerten Lösungsmittel, wie einem Ether oder einem halogenierten Kohlenwasserstoff, z.B. Diethylether, Tetrahydrofuran oder Methylenchlorid, oder in einem Gemisch davon, wenn notwendig, in Gegenwart einer Base, wie einer organischen Base, z.B. eines, vorzugsweise sterisch gehinderten, Triniederalkylamins, z.B. N,N-Diisopropyl-N-ethyl-amin, und unter Kühlen, bei Raumtemperatur oder unter leichtem Erwärmen, z.B. bei etwa -20° C bis etwa 50° C, wenn notwendig, in einem geschlossenen Gefäss und/oder in einer Inertgas-, z.B. Stickstoffatmosphäre.

Eine bevorzugte Ausführungsform der Veresterungsreaktion ist die Umsetzung eines Cäsiumsalzes der Verbindung der Formel III, worin R für Wasserstoff steht, mit dem als Veresterungskomponente gewünschten Alkohol, worin die Hydroxylgruppe in reaktionsfähiger veresterter Form vorliegt, z.B. als Halogenid.

Zur Vermeidung der Lactonbildung arbeitet man entweder mit einem grossen Ueberschuss des als Veresterungskomponente gewünschten Niederalkanols, welcher z.B. gleichzeitig als Lösungsmittel eingesetzt wird, oder man schützt die in der Verbindung der Formel III vorhandenen freien Hydroxylgruppen vor der Veresterung.

Hydroxyschutzgruppen, ihre Einführung und Abspaltung sind beispielsweise beschrieben in "Protective Groups in Organic Chemistry", Plenum Press, London, New York 1973,und in "Methoden der organischen Chemie", Houben-Weyl, 4. Auflage, Bd. 15/1, Georg-Thieme-Verlag, Stuttgart 1974 sowie in Theodora W. Greene, "Protective Groups in Organic Synthesis, John Wiley & Sons, New York 1981. Charakteristisch für Schutzgruppen ist, dass sie leicht, d.h. ohne dass unerwünschte Nebenreaktionen stattfinden, z.B. solvolytisch, reduktiv, photolytisch oder auch unter physiologischen Bedingungen abspaltbar sind.

Hydroxyschutzgruppen sind z.B. Acylreste, wie gegebenenfalls, z.B. durch Halogen, substituiertes Niederalkanoyl, wie 2,2-Dichloracetyl, oder Acylreste von Kohlensäurehalbestern, insbesondere tert.-Butyloxycarbonyl, gegebenenfalls substituiertes Benzyloxycarbonyl, z.B. 4-Nitrobenzyloxycarbonyl, oder Diphenylmethoxycarbonyl, oder 2-Halogen-niederalkoxycarbonyl, wie 2,2,2-Trichlorethoxycarbonyl, ferner Trityl oder Formyl, oder organische Silyl- oder Stannylreste, ferner leicht abspaltbare verethernde Gruppen, wie tert.-Niederalkyl, z.B. tert.-Butyl, 2-oxa- oder 2-thia-aliphatische oder -cycloaliphatische Kohlenwasserstoffreste, in erster Linie 1-Niederalkoxyniederalkyl oder 1-Niederalkylthio-niederalkyl, z.B. Methoxymethyl, 1-Methoxy-ethyl, 1-Ethoxyethyl, Methylthiomethyl, 1-Methylthioethyl oder 1-Ethylthioethyl, oder 2-Oxa- oder 2-Thiacycloalkyl mit 5-6 Ringatomen, z.B. Tetrahydrofuryl oder 2-Tetrahydropyranyl oder entsprechende Thiaanaloge, sowie gegebenenfalls substituiertes 1-Phenylniederalkyl, wie gegebenenfalls substituiertes Benzyl oder Diphenylmethyl, wobei als Substituenten der Phenylreste z.B. Halogen, wie Chlor, Niederalkoxy, wie Methoxy und/oder Nitro in Frage kommen.

Die oben erwähnten organischen Silyl- oder Stannylreste enthalten vorzugsweise Niederalkyl, insbesondere Methyl, als Substituenten der Silizium- oder Zinn-Atome. Entsprechende Silyl-oder Stannylgruppen sind in erster Linie Triniederalkylsilyl, insbesondere Trimethylsilyl, ferner Dimethyl-tert.-butyl-silyl, oder entsprechend substituiertes Stannyl, z.B. Tri-n-butylstannyl.

Die Abspaltung der Hydroxyschutzgruppen erfolgt in an sich bekannter Weise, z.B. mittels Solvolyse, insbesondere Hydrolyse, Alkoholyse oder Acidolyse, oder mittels Reduktion, insbesondere Hydrogenolyse oder chemische Reduktion, gegebenenfalls stufenweise oder gleichzeitig. Triniederalkylsilyl kann auch durch Behandeln mit einem, das Fluoridanion liefernden Salz der Fluorwaserstoffsäure, wie einem Alkalimetallfluorid, z.B. Natrium-oder Kaliumfluorid, in Anwesenheit eines macrocyclischen Polyethers ("Kronenether"), oder mit einem Fluorid einer organischen quaternären Base, wie Tetra-niederalkylammoniumfluorid oder Triniederalkyl-aryl-ammoniumfluorid, z.B. Tetraethylammoniumfluorid oder Tetrabutylammoniumfluorid, in Gegenwart eines aprotischen polaren Lösungsmittels, wie Dimethylsulfoxid oder N,N-Dimethylacetamid, abgespalten werden.

Salze der Verbindung der Formel III, worin R Wasserstoff bedeutet, können in an sich bekannter Weise hergestellt werden, z.B. durch Umsetzung mit einer geeigneten Base, z.B. durch Behandeln mit geeigneten Metallverbindungen, wie Alkalimetallsalzen von geeigneten organischen Carbonsäuren, z.B. dem Natriumsalz der $\alpha$-Ethyl-capronsäure, oder mit geeigneten anorganischen Alkali- oder Erdalkalimetallsalzen, insbesondere solchen, die sich von einer schwachen und vorzugsweise flüchtigen Säure ableiten, z.B. Natriumhydrogencarbonat, oder mit Ammoniak oder einem geeigneten organischen Amin, wobei man vorzugsweise stöchiometrische Mengen oder nur einen kleinen Ueberschuss des salzbildenden Mittels verwendet.

Die Salze können in üblicher Weise in die freie Verbindung übergeführt werden, z.B. durch Behandeln mit geeigneten Säure.

Die oben beschriebenen Verfahren, inklusive die Verfahren zur Abspaltung von Schutzgruppen und die zusätzlichen Verfahrensmassnahmen, werden, wenn nicht anders angegeben, in an sich bekannter Weise, z.B. in Anwesenheit von Lösungs- und Verdünnungsmitteln, wenn notwendig, in Anwesenheit von Kondensationsmitteln oder Katalysatoren, bei erniedrigter oder erhöhter Temperatur, z.B. in einem Temperaturbereich von etwa -20°C bis etwa +100°C, insbesondere von etwa +1°C bis etwa +70°C, hauptsächlich zwischen Raumtemperatur (etwa +20°C) und +45°C, bei Normaldruck oder erhöhtem Druck in einem geeigneten Gefäss und erforderlichenfalls in einer Inertgas-, z.B. Stickstoffatmosphäre, durchgeführt.

Dabei sind unter Berücksichtigung aller im Molekül befindlichen Substituenten, wenn erforderlich, z.B. bei Anwesenheit leicht hydrolysierbarer Reste, besonders schonende Reaktionsbedingungen, wie kurze Reaktionszeiten, Verwendung von milden sauren oder basischen Mitteln in niedriger Konzentration, stöchiometrische Mengenverhältnisse, Wahl geeigneter Katalysatoren, Lösungsmittel, Temperatur- und/oder Druckbedingungen, anzuwenden.

Die Erfindung betrifft auch diejenigen Ausführungsformen des Verfahrens, bei denen man von einer auf irgendeiner Stufe des Verfahrens als Zwischenprodukt erhältlichen Verbindung ausgeht und die fehlenden Verfahrensschritte durchführt oder das Verfahren auf irgendeiner Stufe abbricht oder einen Ausgangsstoff unter den Reaktionsbedingungen bildet oder in Form eines reaktionsfähigen Derivats oder Salzes verwendet. Infolge der engen Beziehung zwischen einer freien Säure und ihren Salzen kann die Erwähnung der freien Säure sinn- und zweckgemäss auch ein Salz davon und umgekehrt bedeuten. Dabei geht man vorzugsweise von solchen Ausgangsstoffen aus, die verfahrensgemäss zu den oben als besonders wertvoll beschriebenen Verbindungen führen.

Neue Ausgangsstoffe und/oder Zwischenprodukte sowie Verfahren zu ihrer Herstellung sind ebenfalls Gegenstand der vorliegenden Erfindung. Vorzugsweise werden solche Ausgangsstoffe verwendet und die Reaktionsbedingungen so gewählt, dass man zu den in dieser Anmeldung als besonders bevorzugt aufgeführten Verbindungen gelangt.

Ausser dem oben für Hormaomycin beschriebenen biologischen und pharmakologischen Wirkungen besitzen Hormaomycin und die Verbindungen der Formel III sowie deren pharmazeutisch verwendbaren Salze antiproliferative, immunsuppressive und entzündungshemmende Eigenschaften, wie sich z.B. aus folgenden Versuchen ergibt:

Zur Demonstration der antiproliferativen Eigenschaften wird die Hemmwirkung der Verbindungen der Formel I auf das Wachstum von menschlichen T24 Blasenkarzinomzellen bestimmt. Diese Zellen werden in "Eagle's minimal essential medium", dem 5 % (V/V) fötales Kälberserum zugesetzt sind, in einem befeuchteten Inkubator bei 37°C und 5 Volumenprozent $CO_2$ in der Luft inkubiert. Die Karzinomzellen (1000-1500) werden in 96-Loch-Mikrotiterplatten überimpft und über Nacht unter den obengenannten Bedingungen inkubiert. Die Testsubstanz wird in seriellen Verdünnungen am Tag 1 hinzugefügt. Die Platten werden unter den obengenannten Bedingungen 5 Tage lang inkubiert. Während dieser Zeitspanne durchlaufen die Kontrollkulturen mindestens 4 Zellteilungen. Nach der Inkubation werden die Zellen in 3,3%iger (G/V) wässriger Glutaraldehydlösung fixiert, mit Wasser gewaschen und mit 0,05%iger (Gewicht/Volumen) wässriger Methylenblaulösung gefärbt. Nach dem Waschen wird der Farbstoff mit 3%iger (G/V) wässriger Salzsäure eluiert. Danach wird die optische Dichte (OD) pro Loch, welche der Zellanzahl direkt proportional ist, mit einem Photometer (Titertek multiskan) bei 665 nm gemessen. Die $IC_{50}$-Werte werden mit einem Computersystem unter Verwendung der Formel

$$\frac{OD_{665}\ (\text{Test}) - OD_{665}\ (\text{Anfang})}{OD_{665}\ (\text{Kontrolle}) - OD_{665}\ (\text{Anfang})} \times 100$$

errechnet. Die $IC_{50}$-Werte sind als diejenige Wirkstoffkonzentration definiert, bei der die Anzahl der Zellen pro Loch am Ende der Inkubationszeit nur 50 % der Zellanzahl in den Kontrollkulturen beträgt. Die so ermittelten $IC_{50}$-Werte liegen für die Verbindungen der Formeln I und III zwischen etwa 0,5 bis 5 µMol/Liter, z.B. bei 1 µMol/Liter.

Die Verbindungen der Formeln I und III eignen sich daher auch zur Therapie von Tumoren, z.B. der Blase.

Die immunsuppressiven Eigenschaften ergeben sich z.B. aus folgenden Versuchen:

<u>Hemmung der durch anti-CD 3 monoklonale Antikörper induzierten T-Zell-Proliferation in menschlichem</u>

Blut

Venöses menschliches Blut wird mit 10 Einheiten Heparin pro ml versetzt und im Verhältnis 1:6 (v:v) mit RPMI 1640 Medium (Gibco) verdünnt. Nach Zugabe von 50 ng/ml anti-CD 3 monoklonalen Antikörpern (TR 66, IgG1K) werden pro Loch 150 $\mu$l verdünntes Blut in eine Lochtiterplatte mit 96 Löchern eingefüllt. Dann werden pro Loch 50 $\mu$l einer Verbindung der Formeln I oder III in dem obengenannten RPMI Medium hinzugefügt. Als Referenzverbindungen verwendet man Cyclosporin A (CSA, Sandoz, Basel, Schweiz) oder Prednisolon.

Nach 48 Stunden bei 37° C und einem Gehalt von 7 Volumenprozent $CO_2$ in der Luft fügt man pro Loch 1.0 $\mu$Ci $^3$H-Thymidin in 10 $\mu$l RPMI Medium (Amersham: spezifische Aktivität 5 Ci/mMol).

Nach weiteren 16 Stunden werden die Zellen mit einer 96-Loch-Erntemaschine (LKB, Bromma, Schweden) auf Glasfiberfiltern (LKB) geerntet. Nach dem Trocknen der Filter wird ihre Radioaktivität gemessen (Optiscint[LKB], Betaplatten-Scintillations-Zähler [LKB]). Die Ergebnisse werden als Durchschnittswerte der für jedes Loch bei drei verschiedenen Konzentrationen in An- und Abwesenheit der Prüfsubstanzen ermittelten cpm (counts pro Minute) ausgedrückt und die Wirkung der Substanzen wird als prozentuale Hemmung angegeben, welche folgendermassen definiert ist:

$$\% \text{ Hemmung} = 100 - \left[ \frac{\text{Exp. cpm}}{\text{cpm der Kontrolle}} \times 100 \right]$$

Exp. cpm = cpm der T-Zellen und der anti-CD 3 monoklonalen Antikörper und der Prüfsubstanz.
cpm der Kontrolle = cpm der T-Zellen und der anti-CD 3 monoklonalen Antikörper (100%ige Inkorporation).

Man zeichnet die Hemmkurve und gibt die $IC_{50}$-Werte in $\mu$Mol/Liter an. Im obengenannten Test werden für Hormaomycin und die Verbindungen der Formel III $IC_{50}$-Werte zwischen 1,5 und 15, z.B. 7,5 $\mu$Mol/Liter und für Cyclosporin A ein $IC_{50}$-Wert von 0,3 $\mu$Mol/Liter ermittelt.

Hemmung der durch Interleukin-2 induzierten T-Zell-Proliferation in menschlichem Blut

Der Versuch wird analog dem vorstehend beschriebenen durchgeführt, wobei an Stelle der 50 ng/ml anti-CD 3 Antikörper 100 Einheiten/ml rekombinantes Interleukin-2 (Hoffmann-La Roche, Basel, Schweiz) zugesetzt werden. In diesem Test werden für Hormaomycin und die Verbindungen der Formel III $IC_{50}$-Werte zwischen 4 und 40, z.B. 17, $\mu$Mol/Liter ermittelt.

Die Verbindungen der Formeln I und III können daher auch als immunsuppressives Mittel zur Unterdrückung unerwünschter Immunreaktionen, z.B. bei Transplantationen oder Allergien, verwendet werden.

Die entzündungshemmenden Eigenschaften können beispielsweise in vitro anhand der Hemmung der Aktivität von Phospholipase $A_2$ (aus menschlichen Leukozyten) gezeigt werden.

In vitro-Test zur Bestimmung der Hemmung von Phospholipase $A_2$ aus menschlichen Leukozyten

Neutrophile polymorphkernige menschliche Leukozyten werden ausgehend von "buffy coats" durch mehrstufige fraktionierte Sedimentation isoliert und tiefgefroren. Die Phospholipase $A_2$ wird aus der Zellsuspension durch Homogenisieren unter Zusatz von eiskalter 0,36 n-$H_2SO_4$ in 2n-NaCl extrahiert und der nach Zentrifugieren bei 10'000 g erhaltene Ueberstand gegen Natriumacetatpuffer pH 4.5 dialysiert.

Für die Bestimmung der Enzymaktivität wird Enzym (10-30 $\mu$g Protein) in 0,1 M Tris/HCl-Puffer pH 7 unter Zusatz von 1 mM $CaCl_2$ und Substrat, bestehend aus biosynthetisch mit $^{14}$C-Oelsäure radioaktiv markierten Phospholipiden (2 $\mu$M) von Escherichia coli bei 37° während 1 Stunde inkubiert. Die Reaktion wird abgestoppt durch Zugabe von Dole-Reagens (Isopropanol/Heptan/1n $H_2SO_4$ 40:10:1, $^v/v$) und die durch Phospholipase $A_2$ selektiv freigesetzte $^{14}$C-Oelsäure extrahiert. Ebenfalls mitextrahiertes Substrat wird durch Filtration des Extraktes durch eine Säule von Kieselgel vollständig entfernt. Die Bestimmung der $^{14}$C-Oelsäure im Eluat erfolgt durch Radiometrie.

Zur Ermittlung der Hemmwirkung der Verbindungen der Formeln I oder III auf die Phospholipase $A_2$

werden diese als Lösungen in Wasser, Dimethylsulfoxid (Endkonzentration im Ansatz bis 5 Vol-%) oder Ethanol (Endkonzentration im Ansatz bis 2.5 Vol-%) dem Inkubationsansatz zugesetzt. Die Wirkungsstärke der Prüfsubstanzen wird ausgedrückt durch die $IC_{50}$, d.h. die Konzentration, welche eine Hemmung von 50 % der Kontrollaktivität bewirkt. Die $IC_{50}$ wird graphisch ermittelt durch Auftragen der prozentualen Hemmung auf der Ordinate gegen den Logarithmus der Konzentration (µM) auf der Abszisse.

In diesem Modell werden für die Verbindungen der Formeln I oder III Werte zwischen 2 und 18, z.B. 7, für die $IC_{50}$ in µMol/Liter erhalten.

Die Erfindung betrifft ebenfalls die Verwendung von Hormaomycin, einer Verbindung der Formel III oder eines pharmazeutisch verwendbaren Salzes der Verbindung der Formel III,

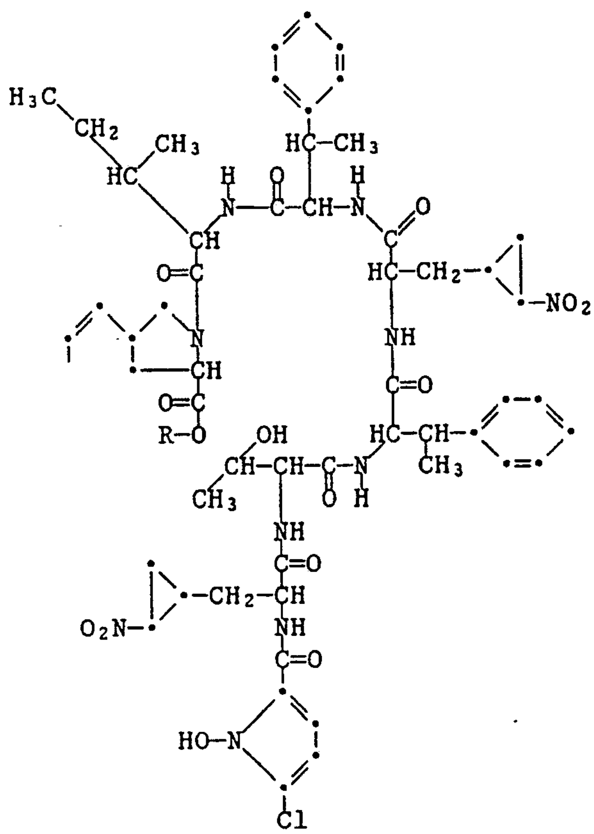

(III)

worin R für Wasserstoff steht, zur medizinischen Behandlung von Krankheiten bei Warmblütern einschliesslich des Menschen, insbesondere durch die Verabreichung therapeutisch wirksamer Mengen der obengenannten Verbindungen. Die Dosierung bei Verabreichung an Warmblüter von etwa 70 kg Körpergewicht, wie z.B. an einen solchen Menschen, hängt unter anderem von der zu behandelnden Krankheit ab und beträgt 0,1 bis 5 g, z.B. 0,5 bis 5 g, vorzugsweise 0,5-2 g, z.B. 1-2 g, pro Tag. Die Verabreichung erfolgt vorzugsweise in Form pharmazeutischer Präparate parenteral, z.B. intravenös, intramuskulär oder intraperitoneal, verteilt auf mehrere Tagesdosen.

Die Erfindung betrifft zudem pharmazeutische Präparate, die Hormaomycin, insbesondere eine effektive, d.h. z.B. antibiotisch wirksame oder tumorhemmende oder unerwünschte Immunreaktionen unterdrückende Dosis von Hormaomycin, einer Verbindung der Formel III oder eines pharmazeutisch verwendbaren Salzes der Verbindung der Formel III, worin R Wasserstoff bedeutet, zusammen mit einem pharmazeutischen Trägermaterial, vorzugsweise mehr als 10 % Trägermaterial, enthalten.

Man kann die pharmakologisch wirksamen Verbindungen der vorliegenden Erfindung in Form von parenteral verabreichbaren Präparaten oder von Infusionslösungen verwenden. Solche Lösungen sind vorzugsweise isotonische wässrige Lösungen oder Suspensionen, wobei diese z.B. bei lyophilisierten Präparaten, welche die Wirksubstanz allein oder zusammen mit einem Trägermaterial, z.B. Mannit, enthalten, vor Gebrauch hergestellt werden können. Die pharmazeutischen Präparate können sterilisiert sein und/oder Hilfsstoffe, z.B. Konservier-, Stabilisier-, Netz- und/oder Emulgiermittel, Löslichkeitsvermittler, Salze zur Regulierung des osmotischen Drucks und/oder Puffer enthalten. Die vorliegenden pharmazeutischen Präparate, die, wenn erwünscht, weitere pharmakologisch wirksame Stoffe, wie Antibiotika, enthalten

können, werden in an sich bekannter Weise, z.B. mittels konventioneller Lösungs- oder Lyophilisierungsverfahren, hergestellt und enthalten von etwa 0,1 % bis 90 % insbesondere von etwa 1 % bis etwa 30 % Aktivstoff(e).

Die nachfolgenden Beispiele illustrieren die Erfindung, ohne sie in irgendeiner Form einzuschränken. Die $R_f$-Werte werden, wenn nicht anders angegeben, auf Kieselgeldünnschichtplatten (Sil G/$UV_{254}$) ermittelt. Das Verhältnis der Laufmittel in den verwendeten Laufmittelgemischen zueinander ist in Volumenanteilen (V/V), Temperaturen sind in Grad Celsius angegeben. Die Konzentration, c, der Substanz im Lösungsmittel(gemisch) ist im Falle der optischen Drehung in Prozent (Gewicht/Volumen) angegeben.

Bei der Angabe der Kernresonanzspektren (chemische Verschiebung $\delta$ in ppm gegenüber Tetramethylsilan) werden folgende Abkürzungen und Kombinationen davon verwendet:

b = breit
d = Dublett
Hz = Hertz
J = Kopplungskonstante
m = Multiplett
q = Quartett
s = Singulett
t = Triplett

Beispiel 1:

Ein 10 Liter Fermenter wird mit 9 Liter Nährlösung (20 g Sojamehl vollfett, 20 g Fleischmehl, 10 g Mannit, 0,2 g NaCl, 0,5 g $ZnSO_4$ x $7H_2O$, 1 Liter Leitungswasser, pH 7,0) beschickt, sterilisiert, mit 1 Liter Vorkultur des Stammes Streptomyces griseoflavus DSM 5195 (gleiche Nährlösung, 32 Stunden alt, 27° C) angeimpft und 45 Stunden unter folgenden Bedingungen betrieben: Temperatur 27° C (nach 8 Stunden absenken auf 20° C), Drehzahl des Rührers 300 Umdrehungen pro Minute, Luftzufuhr 0,5 vvm (vvm = Volumen Luft/Volumen Nährmedium/Minute).

Das durch Zentrifugation der Kulturbrühe gewonnene Mycel wird zweimal mit Methanol extrahiert. Der bis zur wässrigen Phase eingeengte Extrakt wird zweimal mit Essigsäureethylester ausgeschüttelt. Die organische Phase liefert nach Abdampfen des Lösungsmittels ein bräunliches Produkt. Dieses wird an einer Kieselgel-Säule aufgetrennt ($CHCl_3$ als Laufmittel). Die aktiven Fraktionen werden an einer Sephadex®LH-20-Säule (modifiziertes Dextrangel) mit Methanol nachgereinigt. Die Anreicherung von Hormaomycin erfolgt durch Hochdruckflüssigkeitschromatographie (HPLC) (RP hypersil-5 µm, $H_2O/CH_3CN$ (0,1 % $H_3PO_4$) - Gradient: 30 % $CH_3CN$ bis 100 % $CH_3CN$, Detektion: 254 nm, Retentionszeit = 6,7 Minuten (77 % $CH_3CN$)). 140 mg dieser Substanzcharge werden zur Endreinigung an Kieselgel (< 0,08 mm Porendurchmesser; Säule: 2,5 x 50 cm) mit n-Hexan-Aceton (2:1) chromatographiert. Dabei werden 18 mg einer farblosen, mit Molybdatophosphorsäure anfärbbaren (DC) Verunreinigung abgetrennt. Hormaomycin wird mit $CHCl_3$-MeOH (95:5) eluiert. Durch Eintropfen einer konzentrierten Chloroformlösung in 500 ml n-Hexan wird die Substanz umgefällt. Man erhält Hormaomycin als farbloses, amorphes Pulver mit Smp. 166-168°;
Rf = 0,39 ($CHCl_3$:Methanol = 100:4); $R_f$ = 0,28 (n-Hexan:Aceton = 1:1);
$[\alpha]_D^{20}$ = +20,8° (c = 0,5; Methanol); UV (Methanol): 278 (9150), 206 (42950) nm;
IR (KBr): 3390, 3100-2800, 1741, 1645-1620 (breit), 1612, 1545, 1450, 1365 $cm^{-1}$;
Fast atom bombardment-Massenspektrum (Glycerin als Matrix): 1129,4 ($[M+H]^+$), 938, 830, 700, 516, 401, 373, 300, 264, 218;
$^1H$-NMR und $^{13}C$-NMR siehe Tabelle 1;

| | | | | | |
|---|---|---|---|---|---|
| $C_{55}H_{69}N_{10}O_{14}Cl$ (1129,68): Ber. | C 58,48 | H 6,16 | N 12,40 | O 19,82 | Cl 3,14 |
| Gef. | C 58,35 | H 6,31 | N 11,66 | O 19,30 | Cl 2,94 |

Hormaomycin ist neutral, löst sich gut in Methanol, Chloroform, Aceton und Essigsäureethylester und ist in Wasser oder Hexan unlöslich. Auf Dünnschichtchromatographie-Platten löscht es UV-Licht (254 nm), färbt sich beim Stehen an der Luft langsam gelb und lässt sich nach Barrollier (blaugrün) und mit Ehrlich's Reagenz (blass violett), nicht jedoch mit Ninhydrin anfärben.

Tabelle 1: $^{13}C$- und $^{1}H$-NMR-Daten von Hormaomycin ($CDCl_3$, 100,6 bzw. 400 MHz)

| Strukturbestandteil | δ(C) | Qualität[a] | Zuordnung[b] | δ(H) | Aufspaltung | Zuordnung[b] |
|---|---|---|---|---|---|---|
| a-Threonin-Rest | 17,0 | $CH_3$ | C(4) | 1,48 | d, J=7 | $H_3$-C(4) |
| 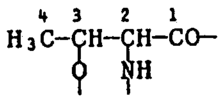 | 55,0 | CH | C(2) | 4,54 | dd, J=9,5/2,5 | H-C(2) |
| | 69,2 | CH | C(3) | 5,35 | dq, J=2,5/7 | H-C(3) |
| | *) | C | C(1) | 8,98 | d, J=9,5 | NH-C(1) |
| Isoleucin-Rest | 10,5 | $CH_3$ | C(5) | 0,82 | t, J=7 | $H_3$-C(5) |
| 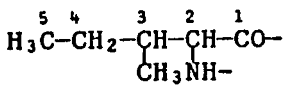 | 15,0 | $CH_3$ | C(6) | 0,97 | d, J=7 | $H_3$-C(6) |
| | 24,9 | $CH_2$ | C(4) | 1,22 | verdeckt | $H_a$-C(4) |
| | | | | 1,49 | verdeckt | $H_b$-C(4) |
| | 37,9 | CH | C(3) | 1,80 | verdeckt | H-C(3) |
| | 54,6 | CH | C(2) | 4,60 | dd, J=9/9 | H-C(2) |
| | *) | C | C(1) | 7,21 | verdeckt | NH-C(1) |
| 3-Methyl-3-phenyl- | 13,4 | $CH_3$ | C(10) | 1,35 | d, J=7 | $H_3$-C(10) |
| alanin-Rest (I) | 39,3 | CH | C(3) | 3,60 | dq, J=7/4,5 | H-C(3) |
| 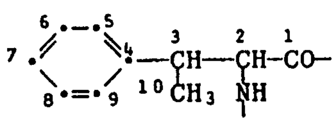 | 60,0 | CH | C(2) | 4,41 | dd, J=9,5/4,5 | H-C(2) |
| | 127,1 | CH | C(7) | 7,08 | m, | H-C(7) |
| | 128,5 | CH (2x) | C(6,8) | 7,15 | m, 4H | H-C(5,6,8,9) |
| | 128,6 | CH (2x) | C(5,9) | | | |
| | 142,1 | C | C(4) | – | | |
| | *) | C | C(1) | 6,85 | d, J=9,5 | NH-C(1) |

Tabelle 1 (Fortsetzung)

| Strukturbestandteil | δ(C) | Qualität[a)] | Zuordnung[b)] | δ(H) | Aufspaltung | Zuordnung[b)] |
|---|---|---|---|---|---|---|
| 3-Methyl-3-phenyl- | 17,9 | $CH_3$ | C(10) | 1,23 | d, J=7 | $H_3$-C(10) |
| alanin-Rest (II) | 41,9 | CH | C(3) | 2,93 | dq, J=10/7 | H-C(3) |
| | 59,7 | CH | C(2) | 4,37 | dd, J=10,5/10,5 | H-C(2) |
| | 126,9 | CH | C(7) | 6,95 | m | |
| | 127,4 | CH (2x) | C(6,8) | 7,06 | m, 4H | H-C(5,6,8,9) |
| | 127,7 | CH (2x) | C(5,9) | | | |
| | 141,6 | C | C(4) | - | | |
| | *) | C | C(1) | 6,77 | verdeckt | NH-C(1) |
| (Z)-4-Propenyl- | 13,4 | $CH_3$ | C(8) | 1,61 | dd, J=7/1,5 | $H_3$-C(8) |
| prolin-Rest | 35,5 | $CH_2$ | C(3) | 1,72 | verdeckt | $H_a$-C(3) |
| 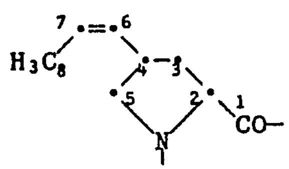 | | | | 2,30 | m | $H_b$-C(3) |
| | 36,7 | CH | C(4) | 3,21 | verdeckt | H-C(4) |
| | 52,8 | $CH_2$ | C(5) | 3,21 | verdeckt | $H_a$-C(5) |
| | | | | 3,91 | m | $H_b$-C(5) |
| | 61,3 | CH | C(2) | 4,20 | dd, J=11,5/6 | H-C(2) |
| | 127,5 | CH | C(6) | 5,20 | m | H-C(6) |
| | 128,2 | CH | C(7) | 5,58 | dq, J=10,5/7 | H-C(7) |
| | *) | C | C(1) | - | | |

Tabelle 1 (Fortsetzung)

| Strukturbestandteil | δ(C) | Qualität[a)] | Zuordnung[b)] | δ(H) | Aufspaltung | Zuordnung[b)] |
|---|---|---|---|---|---|---|
| 3-(2-Nitro-cyclo- | 17,1 | $CH_2$ | C(6) | -0,63 | ddd, J=7/7/7 | $H_a$-C(6) |
| propyl)-alanin-Rest (I) | | | | 0,98 | verdeckt | $H_b$-C(6) |
| 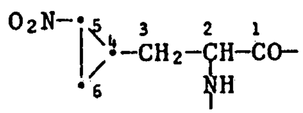 | 20,1 | CH | C(4) | 0,29 | m | H-C(4) |
| | 33,2 | $CH_2$ | C(3) | -0,15 | m | $H_a$-C(3) |
| | | | | 0,48 | m | $H_b$-C(3) |
| | 51,8 | CH | C(2) | 3,48 | m | H-C(2) |
| | 58,2 | CH | C(5) | 2,90 | ddd, J=6,6/3,3/3,3 | H-C(5) |
| | *) | C | C(1) | 6,69 | d, J=9 | NH-C(1) |
| 3-(2-Nitrocyclo-pro- | 17,4 | $CH_2$ | C(6) | 0,98 | verdeckt | $H_a$-C(6) |
| pyl)-alanin-Rest (II) | | | | 1,80 | verdeckt | $H_b$-C(6) |
| | 21,6 | CH | C(4) | 1,85 | verdeckt | H-C(4) |
| | 35,0 | $CH_2$ | C(3) | 1,55 | verdeckt | $H_a$-C(3) |
| | | | | 1,75 | verdeckt | $H_b$-C(3) |
| | 51,0 | CH | C(2) | 5,05 | m | H-C(2) |
| | 59,1 | CH | C(5) | 3,99 | ddd, J=6,6/3,3/3,3 | H-C(5) |
| | *) | C | C(1) | 8,03 | d, J=9 | NH-C(1) |

Tabelle 1 (Fortsetzung)

| Strukturbestandteil | δ(C) | Qualität[a)] | Zuordnung[b)] | δ(H) | Aufspaltung | Zuordnung[b)] |
|---|---|---|---|---|---|---|
| 5-Chlor-N-hydroxy- | 103,5 | CH | C(4) | 6,08 | d, J=4,5 | H-C(4) |
| pyrrol-2-carbonsäure- | 109,7 | CH | C(3) | 6,75 | d, J=4,5 | H-C(3) |
| Rest | 119,7 | C | C(2) | - | | |
| | 121,4 | C | C(5) | - | | |
| 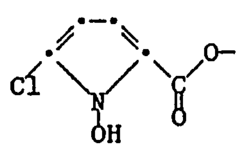 | 159,3 | C | C(6) | 11,10 | br | OH-N(1) |

*) CO-Signale: 168,7 (2x), 169,8, 170,8, 171,3, 171,6 und 171,9

a) Aus APT- und DEPT-Spektren (APT: Attached Proton Test; DEPT: Distorsionless Enhancement by Polarization Transfer)

b) Aus $^{1}H/^{1}H$- und $^{1}H/^{13}C$-COSY-NMR-Spektren

Beispiel 2:

7 mg Hormaomycin werden in 2 ml Methanol gelöst. Nach Zugabe katalytischer Mengen an festem $K_2CO_3$ wird die Lösung 14 Stunden bei Raumtemperatur gerührt, anschliessend auf 20 ml Eiswasser gegossen und mit $CH_2Cl_2$ (4 x 6 ml) extrahiert. Den Verdampfungsrückstand der vereinigten organischen Phasen chromatographiert man an Kieselgel (Säule 1,0 x 3,0 cm, $CHCl_3$:Methanol = 9:1). Aus der Hauptzone erhält man den offenkettigen Hormaomycin-methylester der Formel II; Smp. 172° C, $R_f$ = 0,05 ($CHCl_3$:Methanol = 100:4), $R_f$ = 0,46 ($CHCl_3$:Methanol = 9:1; gleiches Anfärbeverhalten wie Hormaomycin), UV (Methanol): 278 (11200), 214 (31300) nm, IR (KBr): 3400, 3300, 3100-2850, 1740, 1635 (breit), 1545, 1450, 1370, 1030 $cm^{-1}$, Fast atom bombardment-Massenspektrum (2-Nitrobenzylalkohol als Matrix): 1183/1185 ($[M+Na]^+$), 1161/1163 ($[M+H]^+$), 992, 718, 419; 1159/1161 ($[M-H]^-$), $^1H$-NMR ($CDCl_3$, 200 MHz): 3,74 (s, 3H, $OCH_3$), das übrige Signalmuster ist dem Hormaomycin ähnlich, die Signale sind jedoch zum Teil breit und überlappen stärker, es fehlen die Signale jenseits von Tetramethylsilan.

Beispiel 3: Pharmazeutisches Präparat zur intramuskulären Injektion

250 mg Hormaomycin werden in Polyethylenglykol 300 gelöst. Die Lösung wird sterilfiltriert und unter aseptischen Bedingungen in eine 3 ml Ampulle abgefüllt.

## Ansprüche

1. Die Substanz Hormaomycin, welche durch Fermentation mit dem Stamm Streptomyces griseoflavus DSM 5195 erhältlich ist und gegenüber den Stämmen Arthrobacter crystallopoietes ATCC 15481 und Arthrobacter oxydans ATCC 14358 eine minimale Hemmkonzentration aufweist, welche kleiner als 0,01 µg/ml ist.
2. Die Substanz nach Anspruch 1 mit der Formel I.

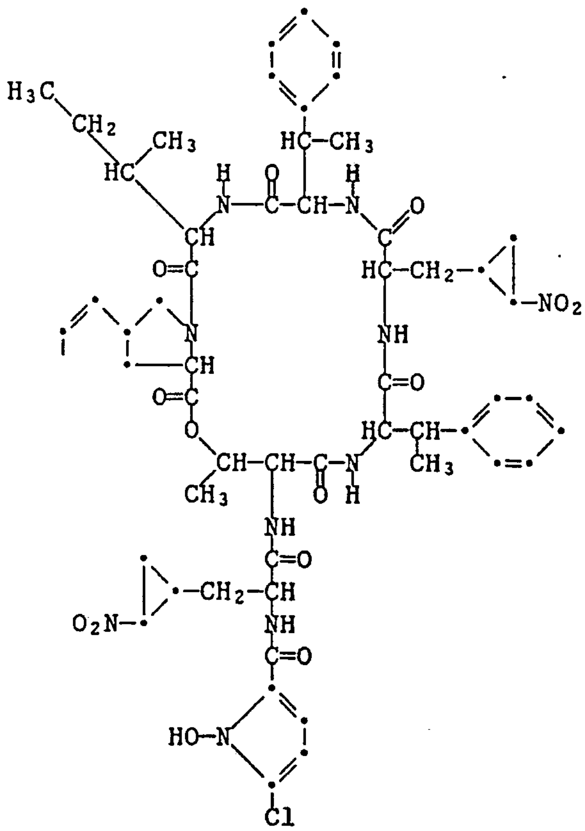

(I)

3. Die Substanz nach Anspruch 1 oder 2, welche innerhalb der Messgenauigkeit mindestens eine der folgenden physikalischen Eigenschaften aufweist:

a) Schmelzpunkt 166-168° C,

b) optische Drehung $[\alpha]_D^{20}$ = +20,8° (c = 0,5; Methanol),

c) Molekulargewicht 1129±2,

d) in $CDCl_3$ als Lösungsmittel $^1$H-NMR-Signale mit einer chemischen Verschiebung $\delta$ gegenüber Tetramethylsilan als interner Standard zwischen +0,5 und -0,7 ppm.

4. Eine aus der Substanz Hormaomycin nach einem der Ansprüche 1-3 durch Lactonspaltung und gegebenenfalls nachfolgende Veresterung mit einem Niederalkanol erhältliche Substanz der Formel III

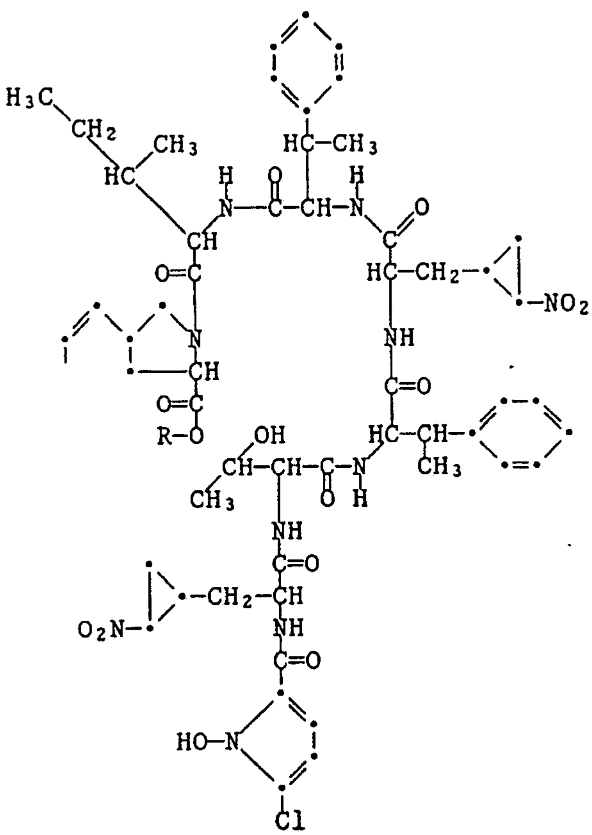

(III)

worin R für Wasserstoff oder Niederalkyl steht, oder ein Salz der Verbindung der Formel III, worin R für Wasserstoff steht.

5. Hormaomycin-methylester nach Anspruch 4.

6. Eine Substanz nach einem der Ansprüche 1-5 oder ein pharmazeutisch verwendbares Salz der Verbindung der Formel III,

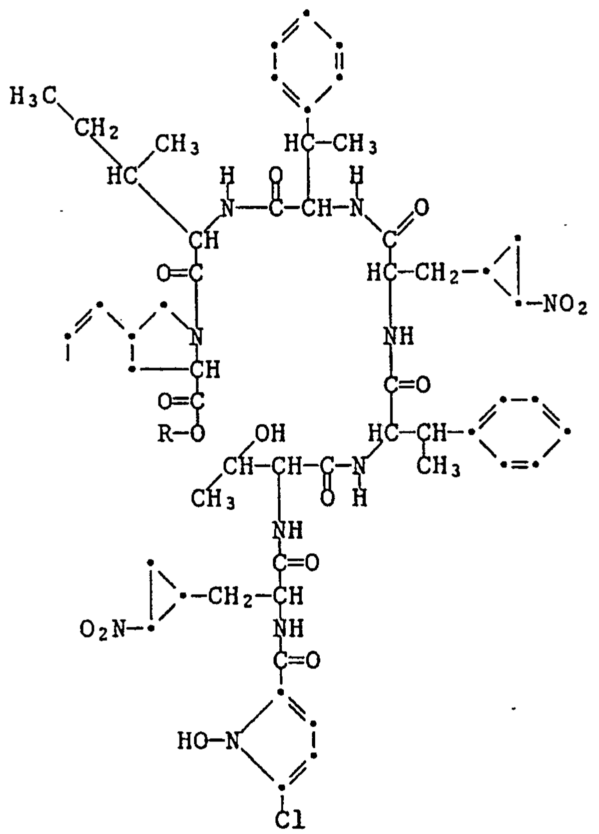

(III)

worin R für Wasserstoff steht, zur Anwendung in einem Verfahren zur therapeutischen Behandlung des menschlichen oder tierischen Körpers.

7. Ein pharmazeutisches Präparat, das eine Substanz nach einem der Ansprüche 1-5 oder ein pharmazeutisch verwendbares Salz der Verbindung der Formel III,

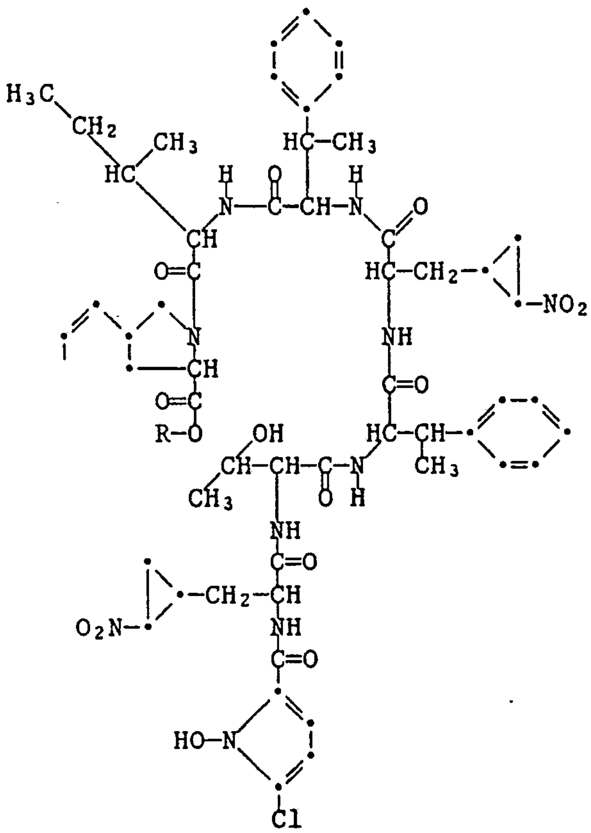

(III)

worin R für Wasserstoff steht, zusammen mit mehr als 10 % an pharmazeutischem Trägermaterial enthält.

8. Verwendung einer Substanz nach einem der Ansprüche 1-5 oder eines pharmazeutisch verwendbaren Salzes der Verbindung der Formel III,

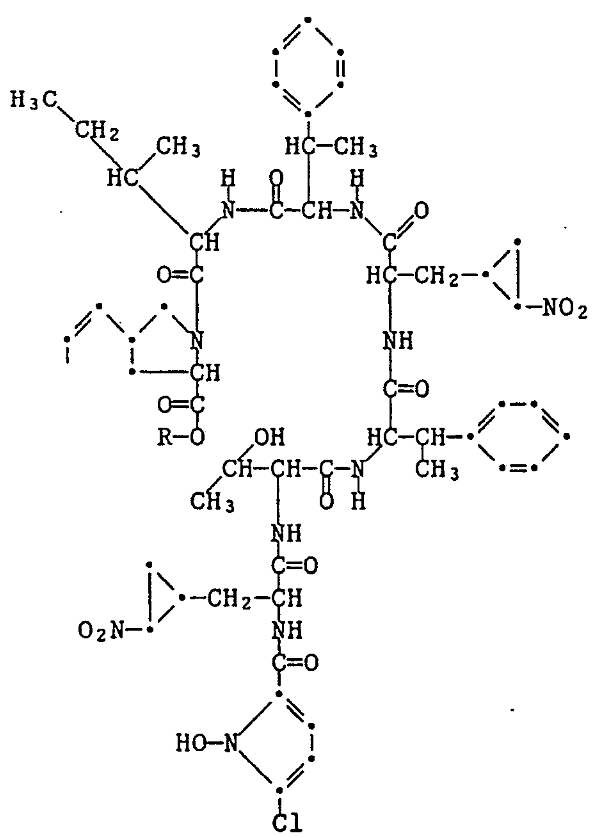

(III)

worin R für Wasserstoff steht, zur Herstellung von Arzneimitteln, die zur Anwendung für die Behandlung von bakteriellen Erkrankungen, für die Unterdrückung unerwünschter Immunreaktionen oder für die Tumortherapie bestimmt sind.

9. Verfahren zur Herstellung von Hormaomycin nach einem der Ansprüche 1-3, dadurch gekennzeichnet, dass man den Stamm Streptomyces griseoflavus DSM 5195 oder eine von diesem Stamm ableitbare Hormaomycin bildende Kultur in einem geeigneten Nährmedium züchtet und Hormaomycin aus der Kulturbrühe isoliert.

10. Der Stamm Streptomyces griseoflavus DSM 5195 und davon abgeleitete Kulturen, die zur Produktion von Hormaomycin befähigt sind.

Patentansprüche für folgende Vertragsstaaten: ES,GR

1. Verfahren zur Herstellung der Substanz Hormaomycin, welche gegenüber den Stämmen Arthrobacter crystallopoietes ATCC 15481 und Arthrobacter oxydans ATCC 14358 eine minimale Hemmkonzentration aufweist, welche kleiner als 0,01 µg/ml ist, wobei Hormaomycin wahrscheinlich eine Struktur gemäss Formel I

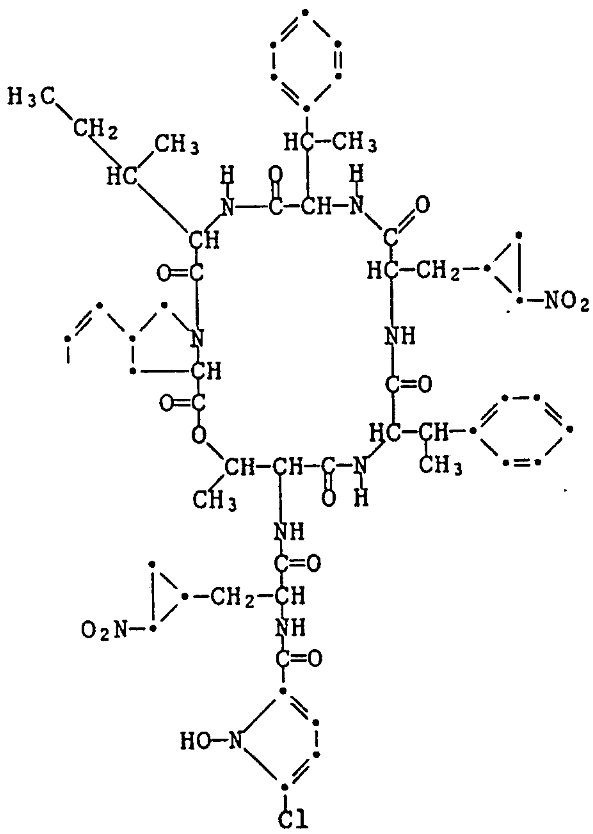

(I)

und innerhalb der Messgenauigkeit die folgenden physikalischen Eigenschaften aufweist:

a) Schmelzpunkt 166-168° C,

b) optische Drehung $[a]_D^{20}$ = +20,8° (c = 0,5; Methanol),

c) Molekulargewicht 1129±2,

d) in $CDCl_3$ als Lösungsmittel $^1H$-NMR-Signale mit einer chemischen Verschiebung $\delta$ gegenüber Tetramethylsilan als internem Standard zwischen +0,5 und -0,7 ppm,

dadurch gekennzeichnet, dass man den Stamm Streptomyces griseoflavus DSM 5195 oder eine von diesem Stamm ableitbare Hormaomycin bildende Kultur in einem geeigneten Nährmedium züchtet und Hormaomycin aus der Kulturbrühe isoliert.

2. Verfahren nach Anspruch 1, dadurch gekennzeichnet, dass man den Stamm Streptomyces griseoflavus DSM 5195 oder einen Mikroorganismus, der die gleichen Strukturgene enthält wie die für die Bildung von Hormaomacin ursächlichen Strukturgene des genannten Stammes, in einem wässerigen, eine Kohlenstoff- und Stickstoffquelle sowie anorganische Salze enthaltenden Nährmedium aerob züchtet und Hormaomycin isoliert.

3. Verfahren nach Anspruch 1, dadurch gekennzeichnet, dass man einen Hormaomycin bildenden Mikroorganismus der Art Streptomyces griseoflavus züchtet.

4. Verfahren nach Anspruch 1, dadurch gekennzeichnet, dass man den Stamm Streptomyces griseoflavus DSM 5195 oder eine Hormaomycin bildende Mutante dieses Stammes züchtet.

5. Verfahren nach Anspruch 1, dadurch gekennzeichnet, dass man den Stamm Streptomyces griseoflavus DSM 5195 züchtet.

6. Verfahren zur Weiterverarbeitung von Hormaomycin unter Herstellung einer Verbindung der Formel III,

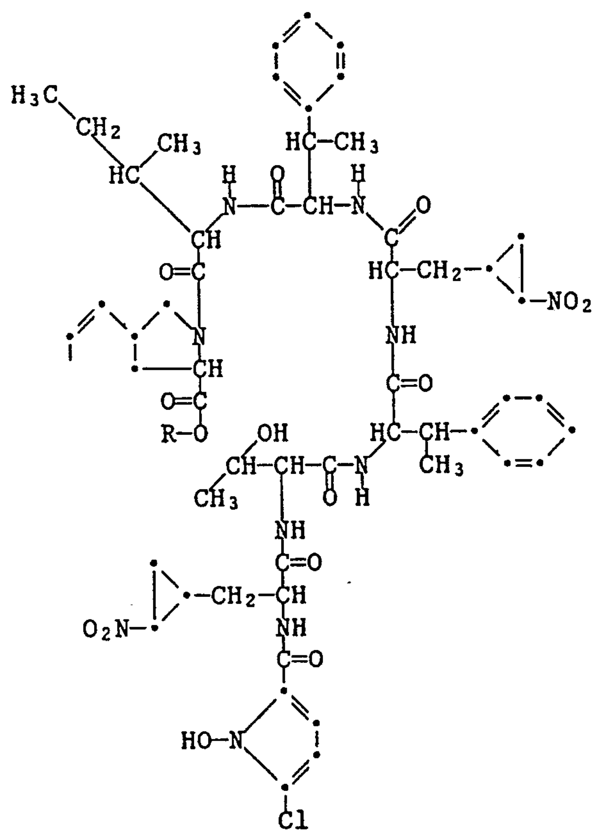

(III)

worin R für Wasserstoff oder Niederalkyl steht, oder eines Salzes der Verbindung der Formel III, worin R für Wasserstoff steht, dadurch gekennzeichnet, dass man Hormaomycin der Formel I

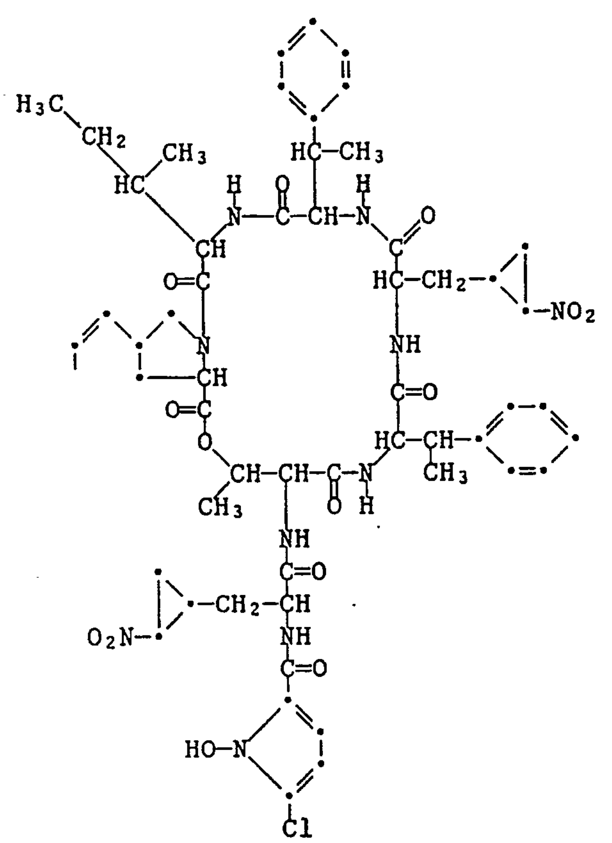

(I)

einer Lactonspaltung unterzieht, wodurch man eine Verbindung der Formel III, worin R für Wasserstoff steht, oder ein Salz davon erhält, und zur Herstellung einer Verbindung der Formel III, worin R für Niederalkyl steht, gegebenenfalls die Verbindung der Formel III, worin R für Wasserstoff steht, erforderlichenfalls nach Schutz der freien Hydroxylgruppen durch leicht abspaltbare Schutzgruppen, verestert und vorhandene Schutzgruppen abspaltet, oder zur Herstellung eines Salzes die Verbindung der Formel III, worin R Wasserstoff bedeutet, in ein Salz überführt oder ein erhaltenes Salz in die freie Säure umwandelt.

7. Verfahren nach Anspruch 6 zur Herstellung einer Verbindung der Formel III, worin R für Methyl steht, dadurch gekennzeichnet, dass man Hormaomycin in Methanol löst und mit katalytischen Mengen einer geeigneten schwachen Base behandelt und die gewünschte Verbindung aus dem Reaktionsgemisch isoliert.

8. Verfahren zur Herstellung von pharmazeutischen Präparaten, dadurch gekennzeichnet, dass man in die genannten Präparate eine gemäss einem der Ansprüche 1 bis 7 erhaltene Verbindung in einem Prozentsatz von 1 bis 30 % einarbeitet.

Europäisches Patentamt

# EUROPÄISCHER RECHERCHENBERICHT

Nummer der Anmeldung

EP 90810121.5

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| P,X | ANGEWANDTE CHIEMIE, Vol. 102, Nr. 1, Jänner 1990, Weinheim E.RÖSSNER et al. "Aufklärung der Struktur von Hormaomycin" Seiten 84-85 * Gesamt * -- | 1-7 | C 12 P 21/00<br>C 12 P 1/06<br>A 61 K 37/02<br>//(C 12 P 1/06<br>C 12 R 1:465) |
| P,X | HELVETICA CHIMICA ACTA, Vol. 72, Fasciculus 3, 3. Mai 1989, Basel N.ANDRES et al. "Hormaomycin, ein neues Peptidlacton mit morphogener Aktivität auf Streptomyceten" Seiten 426-437 * Gesamt * ---- | 1 | |
| | | | RECHERCHIERTE SACHGEBIETE (Int. Cl.5) |
| | | | C 12 P<br>A 61 K |

Der vorliegende Recherchenbericht wurde für alle Patentansprüche erstellt.

| Recherchenort | Abschlußdatum der Recherche | Prüfer |
|---|---|---|
| WIEN | 04-06-1990 | WOLF |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument